# EUROPEAN PATENT SPECIFICATION

(11) **EP 2 635 593 B1**
(45) Date of publication and mention of the grant of the patent: **14.09.2016**
(21) Application number: 11778646.7
(22) Date of filing: 04.11.2011
(51) Int. Cl.: A61K 39/095, C07K 14/22, C12N 15/62, C07K 19/00

(54) **VACCINES FOR PREVENTING MENINGOCOCCAL INFECTIONS**
IMPFSTOFFE ZUR VERHINDERUNG VON MENINGOKOKKEN-INFEKTIONEN
VACCINS POUR LA PRÉVENTION D'INFECTIONS À MÉNINGOCOQUE

(30) Priority: 05.11.2010 EP 10306215
(43) Date of publication of application: 11.09.2013
(73) Proprietor: Institut National de la Santé et de la Recherche Médicale (INSERM), 75013 Paris (FR); Université Paris Descartes, 75270 Paris Cedex 06 (FR)
(72) Inventor: COUREUIL, Mathieu, 75730 Paris Cedex 15 (FR); NASSIF, Xavier, 75730 Paris Cedex 15 (FR); MARULLO, Stefano, 75014 Paris (FR)
(74) Representative: Lavoix
(86) International application number: PCT/EP2011/069463
(87) International publication number: WO 2012/059593

(56) References cited:
- WO-A1-94/08013
- WO-A2-99/55875
- WO-A2-03/060120
- CEHOVIN A ET AL: "Sequence conservation of pilus subunits in Neisseria meningitidis", VACCINE, ELSEVIER LTD, GB, vol. 28, no. 30, 5 July 2010 (2010-07-05), pages 4817-4826, XP027118999, ISSN: 0264-410X [retrieved on 2010-07-02]
- JOIN-LAMBERT O ET AL: "Mechanisms of meningeal invasion by a bacterial extracellular pathogen, the example of Neisseria meningitidis", PROGRESS IN NEUROBIOLOGY, PERGAMON PRESS, GB, vol. 91, no. 2, 1 June 2010 (2010-06-01), pages 130-139, XP027021185, ISSN: 0301-0082 [retrieved on 2010-04-22]
- MIKATY GUILLAIN ET AL: "Extracellular Bacterial Pathogen Induces Host Cell Surface Reorganization to Resist Shear Stress", PLOS PATHOGENS, vol. 5, no. 2, E1000314, February 2009 (2009-02), pages 1-14, XP002631839, cited in the application
- ORIHUELA CARLOS J ET AL: "Laminin receptor initiates bacterial contact with the blood brain barrier in experimental meningitis models", JOURNAL OF CLINICAL INVESTIGATION, vol. 119, no. 6, June 2009 (2009-06), pages 1638-1646, XP002631840, ISSN: 0021-9738
- J. DONNELLY ET AL: "Qualitative and quantitative assessment of meningococcal antigens to evaluate the potential strain coverage of protein-based vaccines", PROCEEDINGS OF THE NATIONAL ACADEMY OF SCIENCES, vol. 107, no. 45, 20 October 2010 (2010-10-20), pages 19490-19495, XP055002659, ISSN: 0027-8424, DOI: 10.1073/pnas.1013758107
- COUREUIL MATHIEU ET AL: "Meningococcus Hijacks a beta 2-Adrenoceptor/beta-Arrestin Pathway to Cross Brain Microvasculature Endothelium", CELL, vol. 143, no. 7, December 2010 (2010-12), pages 1149-1160, XP002631841, ISSN: 0092-8674

### SEQUENCE LISTING

<110> INSTITUT NATIONAL DE LA SANTE ET DE LA RECHERCHE MEDICALE (INSERM)
<120> VACCINES FOR PREVENTING MENINGOCOCCAL INFECTIONS
<130> BET11P2899
<150> EP 10306215.4
   <151> 2010-11-05
<160> 30
<170> PatentIn version 3.5
<210> 1
   <211> 168
   <212> PRT
   <213> Neisseria meningitidis
<400> 1
   Met Asn Thr Leu Gln Lys Gly Phe Thr Leu Ile Glu Leu Met Ile Val
   1               5                   10                  15
   Ile Ala Ile Val Gly Ile Leu Ala Ala Val Ala Leu Pro Ala Tyr Gln
               20                  25                  30
   Asp Tyr Thr Ala Arg Ala Gln Val Ser Glu Ala Ile Leu Leu Ala Glu
           35                  40                  45
   Gly Gln Lys Ser Ala Val Thr Glu Tyr Tyr Leu Asn His Gly Glu Trp
       50                  55                  60
   Pro Gly Asp Asn Ser Ser Ala Gly Val Ala Thr Ser Ala Asp Ile Lys
   65                  70                  75                  80
   Gly Lys Tyr Val Gln Ser Val Thr Val Ala Asn Gly Val Ile Thr Ala
                   85                  90                  95
   Gln Met Ala Ser Ser Asn Val Asn Asn Glu Ile Lys Ser Lys Lys Leu
               100                 105                 110
   Ser Leu Trp Ala Lys Arg Gln Asn Gly Ser Val Lys Trp Phe Cys Gly
           115                 120                 125
   Gln Pro Val Thr Arg Thr Thr Ala Thr Ala Thr Asp Val Ala Ala Ala
       130                 135                 140
   Asn Gly Lys Thr Asp Asp Lys Ile Asn Thr Lys His Leu Pro Ser Thr
   145                 150                 155                 160
   Cys Arg Asp Asp Ser Ser Ala Ser
                   165
<210> 2
   <211> 129
   <212> PRT
   <213> Neisseria meningitidis
<400> 2
   Met Lys Asn Val Gln Lys Gly Phe Thr Leu Leu Glu Leu Met Ile Ala
   1               5                   10                  15
   Val Ala Ile Leu Gly Ile Leu Thr Leu Ile Thr Tyr Pro Ser Tyr Lys
               20                  25                  30
   Thr Tyr Ile Arg Arg Val Arg Leu Ser Glu Val Arg Thr Thr Leu Leu
           35                  40                  45
   His Asn Ala Gln Thr Met Glu Arg Tyr Tyr Arg Gln Lys Gly Thr Phe
       50                  55                  60
   Lys Thr Tyr Asp Lys Asn Lys Leu Lys Gln Asn Lys Tyr Phe Asn Val
   65                  70                  75                  80
   Thr Leu Ser Lys Val Ser Pro Asp His Phe Thr Leu Gln Ala Asp Pro
                   85                  90                  95
   Asn Pro Thr Thr Asn Asp Gly Glu Thr Cys Val Val Thr Leu Asn Asp
               100                 105                 110
   Gly Gly Thr Ile Ala Ala Ser Gly Thr Asn Gln Ser Cys Pro Gly Phe
           115                 120                 125
   Asp
<210> 3
   <211> 413
   <212> PRT
   <213> Homo sapiens
<220>
   <221> DOMAIN
   <222> (1)..(28)
   <223> N-terminus
<400> 3
<210> 4
   <211> 21
   <212> RNA
   <213> Artificial
<220>
   <223> siRNA
<400> 4
   aaagccuucu gcgcggagaa u 21
<210> 5
   <211> 21
   <212> RNA
   <213> Artificial
<220>
   <223> siRNA
<400> 5
   ggcuuguccu uccgcaaaga c 21
<210> 6
   <211> 23
   <212> RNA
   <213> Artificial
<220>
   <223> siRNA
<400> 6
   aagaaguacg agaagcugga guu 23
<210> 7
   <211> 21
   <212> DNA
   <213> Artificial
<220>
   <223> offtarget siRNA
<400> 7
   gataaggtgc tgcgtggacc c 21
<210> 8
   <211> 25
   <212> DNA
   <213> Artificial
<220>
   <223> primer
<400> 8
   gaattccggc gcgtccgcct gtcgg 25
<210> 9
   <211> 26
   <212> DNA
   <213> Artificial
<220>
   <223> primer
<400> 9
   ctgcagttag tcgaagccgg ggcagg 26
<210> 10
   <211> 26
   <212> DNA
   <213> Artificial
<220>
   <223> primer
<400> 10
   gaattcgaga aagcaaagat aaatgc 26
<210> 11
   <211> 28
   <212> DNA
   <213> Artificial
<220>
   <223> primer
<400> 11
   ctgcagctac ttaaataact tgcagtcc 28
<210> 12
   <211> 20
   <212> DNA
   <213> Artificial
<220>
   <223> primer
<400> 12
   attgagaccc tgtgcgtgat 20
<210> 13
   <211> 20
   <212> DNA
   <213> Artificial
<220>
   <223> primer
<400> 13
   ctgaaagacc ctggagtaga 20
<210> 14
   <211> 20
   <212> DNA
   <213> Artificial
<220>
   <223> primer
<400> 14
   ttcctacccc caatgtgtcc 20
<210> 15
   <211> 20
   <212> DNA
   <213> Artificial
<220>
   <223> primer
<400> 15
   tcttgctcag tgtccttgct 20
<210> 16
   <211> 52
   <212> PRT
   <213> Neisseria meningitidis
<400> 16
   Phe Thr Leu Ile Glu Leu Met Ile Val Ile Ala Ile Val Gly Ile Leu
   1               5                   10                  15
   Ala Ala Val Ala Leu Pro Ala Tyr Gln Asp Tyr Thr Ala Arg Ala Gln
               20                  25                  30
   Val Ser Glu Ala Ile Leu Leu Ala Glu Gly Gln Lys Ser Ala Val Thr
           35                  40                  45
   Glu Tyr Tyr Leu
       50
<210> 17
   <211> 53
   <212> PRT
   <213> Neisseria meningitidis
<400> 17
   Phe Thr Leu Leu Glu Leu Met Ile Ala Val Ala Ile Leu Gly Ile Leu
   1               5                   10                  15
   Thr Leu Ile Thr Tyr Pro Ser Tyr Lys Thr Tyr Ile Arg Arg Val Arg
               20                  25                  30
   Leu Ser Glu Val Arg Thr Thr Leu Leu His Asn Ala Gln Thr Met Glu
           35                  40                  45
   Arg Tyr Tyr Arg Gln
       50
<210> 18
   <211> 117
   <212> PRT
   <213> Neisseria meningitidis
<400> 18
   Ser Ala Val Thr Glu Tyr Tyr Leu Asn His Gly Glu Trp Pro Gly Asp
   1 5 10 15
   Asn Ser Ser Ala Gly Val Ala Thr Ser Ala Asp Ile Lys Gly Lys Tyr
   20 25 30
   Val Gln Ser Val Thr Val Ala Asn Gly Val Ile Thr Ala Gln Met Ala
   35 40 45
   Ser Ser Asn Val Asn Asn Glu Ile Lys Ser Lys Lys Leu Ser Leu Trp
   50 55 60
   Ala Lys Arg Gln Asn Gly Ser Val Lys Trp Phe Cys Gly Gln Pro Val
   65 70 75 80
   Thr Arg Thr Thr Ala Thr Ala Thr Asp Val Ala Ala Ala Asn Gly Lys
   85 90 95
   Thr Asp Asp Lys Ile Asn Thr Lys His Leu Pro Ser Thr Cys Arg Asp
   100 105 110
   Asp Ser Ser Ala Ser
   115
<210> 19
   <211> 76
   <212> PRT
   <213> Neisseria meningitidis
<400> 19
   Thr Met Glu Arg Tyr Tyr Arg Gln Lys Gly Thr Phe Lys Thr Tyr Asp
   1 5 10 15
   Lys Asn Lys Leu Lys Gln Asn Lys Tyr Phe Asn Val Thr Leu Ser Lys
   20 25 30
   Val Ser Pro Asp His Phe Thr Leu Gln Ala Asp Pro Asn Pro Thr Thr
   35 40 45
   Asn Asp Gly Glu Thr Cys Val Val Thr Leu Asp Gly Gly Thr Ile Ala
   50 55 60
   Ala Ser Gly Thr Asn Gln Ser Cys Pro Gly Phe Asp
   65 70 75
<210> 20
   <211> 35
   <212> PRT
   <213> Neisseria meningitidis
<400> 20
   Cys Gly Gln Pro Val Thr Arg Thr Thr Ala Thr Ala Thr Asp Val Ala
   1               5                   10                  15
   Ala Ala Asn Gly Lys Thr Asp Asp Lys Ile Asn Thr Lys His Leu Pro
               20                  25                  30
   Ser Thr Cys
           35
<210> 21
   <211> 27
   <212> PRT
   <213> Neisseria meningitidis
<400> 21
   Gly Glu Thr Cys Val Val Thr Leu Asn Asp Gly Gly Thr Ile Ala Ala
   1               5                   10                  15
   Ser Gly Thr Asn Gln Ser Cys Pro Gly Phe Asp
               20                  25
<210> 22
   <211> 280
   <212> PRT
   <213> Neisseria meningitidis
<400> 22
   Met Thr Arg Ser Lys Pro Val Asn Arg Thr Ala Phe Cys Cys Leu Ser
   1               5                   10                  15
   Leu Thr Ala Ala Leu Ile Leu Thr Ala Cys Ser Ser Gly Gly Gly Gly
               20                  25                  30
   Val Ala Ala Asp Ile Gly Ala Val Leu Ala Asp Ala Leu Thr Ala Pro
           35                  40                  45
   Leu Asp His Lys Asp Lys Ser Leu Gln Ser Leu Thr Leu Asp Gln Ser
       50                  55                  60
   Val Arg Lys Asn Glu Lys Leu Lys Leu Ala Ala Gln Gly Ala Glu Lys
   65                  70                  75                  80
   Thr Tyr Gly Asn Gly Asp Ser Leu Asn Thr Gly Lys Leu Lys Asn Asp
                   85                  90                  95
   Lys Val Ser Arg Phe Asp Phe Ile Arg Gln Ile Glu Val Asp Gly Gln
               100                 105                 110
   Leu Ile Thr Leu Glu Ser Gly Glu Phe Gln Val Tyr Lys Gln Ser His
           115                 120                 125
   Ser Ala Leu Thr Ala Leu Gln Thr Glu Gln Val Gln Asp Ser Glu His
       130                 135                 140
   Ser Gly Lys Met Val Ala Lys Arg Gln Phe Arg Ile Gly Asp Ile Ala
   145                 150                 155                 160
   Gly Glu His Thr Ser Phe Asp Lys Leu Pro Glu Gly Gly Arg Ala Thr
                   165                 170                 175
   Tyr Arg Gly Thr Ala Phe Gly Ser Asp Asp Ala Ser Gly Lys Leu Thr
               180                 185                 190
   Tyr Thr Ile Asp Phe Ala Ala Lys Gln Gly His Gly Lys Ile Glu His
           195                 200                 205
   Leu Lys Ser Pro Glu Leu Asn Val Asp Leu Ala Ala Ser Asp Ile Lys
       210                 215                 220
   Pro Asp Lys Lys Arg His Ala Val Ile Ser Gly Ser Val Leu Tyr Asn
   225                 230                 235                 240
   Gln Ala Glu Lys Gly Ser Tyr Ser Leu Gly Ile Phe Gly Gly Gln Ala
   245 250 255
   Gln Glu Val Ala Gly Ser Ala Glu Val Glu Thr Ala Asn Gly Ile Arg
   260 265 270
   His Ile Gly Leu Ala Ala Lys Gln
   275 280
<210> 23
   <211> 279
   <212> PRT
   <213> Neisseria meningitidis
<400> 23
   Met Thr Arg Ser Lys Pro Val Asn Arg Thr Ala Phe Cys Cys Leu Ser
   1               5                   10                  15
   Leu Thr Ala Ala Leu Ile Leu Thr Ala Cys Ser Ser Gly Gly Gly Gly
               20                  25                  30
   Val Ala Ala Asp Ile Gly Ala Gly Leu Ala Asp Ala Leu Thr Ala Pro
           35                  40                  45
   Leu Asp His Lys Asp Lys Ser Leu Gln Ser Leu Thr Leu Asp Gln Ser
       50                  55                  60
   Val Arg Lys Asn Glu Lys Leu Lys Leu Ala Ala Gln Gly Ala Glu Lys
   65                  70                  75                  80
   Thr Tyr Gly Asn Gly Asp Ser Leu Asn Thr Gly Lys Leu Lys Asn Asp
                   85                  90                  95
   Lys Val Ser Arg Phe Asp Phe Ile Arg Gln Ile Glu Val Asp Gly Gln
               100                 105                 110
   Leu Ile Thr Leu Glu Ser Gly Glu Phe Gln Ile Tyr Lys Gln Asp His
           115                 120                 125
   Ser Ala Val Val Ala Leu Gln Ile Glu Lys Ile Asn Asn Pro Asp Lys
       130                 135                 140
   Ile Asp Ser Leu Ile Asn Gln Arg Ser Phe Leu Val Ser Gly Leu Gly
   145                 150                 155                 160
   Gly Glu His Thr Ala Phe Asn Gln Leu Pro Gly Gly Lys Ala Glu Tyr
                   165                 170                 175
   His Gly Lys Ala Phe Ser Ser Asp Asp Ala Gly Gly Lys Leu Thr Tyr
               180                 185                 190
   Thr Ile Asp Phe Ala Ala Lys Gln Gly His Gly Lys Ile Glu His Leu
           195                 200                 205
   Lys Thr Pro Glu Gln Asn Val Glu Leu Ala Ala Ala Glu Leu Lys Ala
       210                 215                 220
   Asp Glu Lys Ser His Ala Val Ile Leu Gly Asp Thr Arg Tyr Gly Ser
   225                 230                 235                 240
   Glu Glu Lys Gly Thr Tyr His Leu Ala Leu Phe Gly Asp Arg Ala Gln
                   245                 250                 255
   Glu Ile Ala Gly Ser Ala Thr Val Lys Ile Gly Glu Lys Val His Glu
               260                 265                 270
   Ile Gly Ile Ala Gly Lys Gln
           275
<210> 24
   <211> 395
   <212> PRT
   <213> Neisseria meningitidis
<400> 24
   Met Arg Lys Lys Leu Thr Ala Leu Val Leu Ser Ala Leu Pro Leu Ala
   1               5                   10                  15
   Ala Val Ala Asp Val Ser Leu Tyr Gly Glu Ile Lys Ala Gly Val Glu
               20                  25                  30
   Gly Arg Asn Tyr Gln Leu Gln Leu Thr Glu Ala Gln Ala Ala Asn Gly
           35                  40                  45
   Gly Ala Ser Gly Gln Val Lys Val Thr Lys Val Thr Lys Ala Lys Ser
       50                  55                  60
   Arg Ile Arg Thr Lys Ile Ser Asp Phe Gly Ser Phe Ile Gly Phe Lys
   65                  70                  75                  80
   Gly Ser Glu Asp Leu Gly Glu Gly Leu Lys Ala Val Trp Gln Leu Glu
                   85                  90                  95
   Gln Asp Val Ser Val Ala Gly Gly Gly Ala Thr Gln Trp Gly Asn Arg
               100                 105                 110
   Glu Ser Phe Ile Gly Leu Ala Gly Glu Phe Gly Thr Leu Arg Ala Gly
           115                 120                 125
   Arg Val Ala Asn Gln Phe Asp Asp Ala Ser Gln Ala Ile Asp Pro Trp
       130                 135                 140
   Asp Ser Asn Asn Asp Val Ala Ser Gln Leu Gly Ile Phe Lys Arg His
   145                 150                 155                 160
   Asp Asp Met Pro Val Ser Val Arg Tyr Asp Ser Pro Glu Phe Ser Gly
                   165                 170                 175
   Phe Ser Gly Ser Val Gln Phe Val Pro Ala Gln Asn Ser Lys Ser Ala
               180                 185                 190
   Tyr Lys Pro Ala Tyr Trp Thr Thr Val Asn Thr Gly Ser Ala Thr Thr
           195                 200                 205
   Thr Thr Phe Val Pro Ala Val Val Gly Lys Pro Gly Ser Asp Val Tyr
       210                 215                 220
   Tyr Ala Gly Leu Asn Tyr Lys Asn Gly Gly Phe Ala Gly Asn Tyr Ala
   225                 230                 235                 240
   Phe Lys Tyr Ala Arg His Ala Asn Val Gly Arg Asp Ala Phe Glu Leu
                   245                 250                 255
   Phe Leu Leu Gly Ser Gly Ser Asp Gln Ala Lys Gly Thr Asp Pro Leu
               260                 265                 270
   Lys Asn His Gln Val His Arg Leu Thr Gly Gly Tyr Glu Glu Gly Gly
           275                 280                 285
   Leu Asn Leu Ala Leu Ala Ala Gln Leu Asp Leu Ser Glu Asn Gly Asp
       290                 295                 300
   Lys Thr Lys Asn Ser Thr Thr Glu Ile Ala Ala Thr Ala Ser Tyr Arg
   305                 310                 315                 320
   Phe Gly Asn Ala Val Pro Arg Ile Ser Tyr Ala His Gly Phe Asp Phe
                   325                 330                 335
   Ile Glu Arg Gly Lys Lys Gly Glu Asn Thr Ser Tyr Asp Gln Ile Ile
               340                 345                 350
   Ala Gly Val Asp Tyr Asp Phe Ser Lys Arg Thr Ser Ala Ile Val Ser
           355                 360                 365
   Gly Ala Trp Leu Lys Arg Asn Thr Gly Ile Gly Asn Tyr Thr Gln Ile
       370                 375                 380
   Asn Ala Ala Ser Val Gly Leu Arg His Lys Phe
   385                 390                 395
<210> 25
   <211> 497
   <212> PRT
   <213> Neisseria meningitidis
<400> 25
   Met Phe Lys Arg Ser Val Ile Ala Met Ala Cys Ile Val Ala Leu Ser
   1               5                   10                  15
   Ala Cys Gly Gly Gly Gly Gly Gly Ser Pro Asp Val Lys Ser Ala Asp
               20                  25                  30
   Thr Leu Ser Lys Pro Ala Ala Pro Val Val Thr Glu Asp Val Gly Glu
           35                  40                  45
   Glu Val Leu Pro Lys Glu Lys Lys Asp Glu Glu Ala Val Ser Gly Ala
       50                  55                  60
   Pro Gln Ala Asp Thr Gln Asp Ala Thr Ala Gly Lys Gly Gly Gln Asp
   65                  70                  75                  80
   Met Ala Ala Val Ser Ala Glu Asn Thr Gly Asn Gly Gly Ala Ala Thr
                   85                  90                  95
   Thr Asp Asn Pro Glu Asn Lys Asp Glu Gly Pro Gln Asn Asp Met Pro
               100                 105                 110
   Gln Asn Ala Ala Asp Thr Asp Ser Ser Thr Pro Asn His Thr Pro Ala
           115                 120                 125
   Pro Asn Met Pro Thr Arg Asp Met Gly Asn Gln Ala Pro Asp Ala Gly
       130                 135                 140
   Glu Ser Ala Gln Pro Ala Asn Gln Pro Asp Met Ala Asn Ala Ala Asp
   145                 150                 155                 160
   Gly Met Gln Gly Asp Asp Pro Ser Ala Gly Glu Asn Ala Gly Asn Thr
                   165                 170                 175
   Ala Asp Gln Ala Ala Asn Gln Ala Glu Asn Asn Gln Val Gly Gly Ser
               180                 185                 190
   Gln Asn Pro Ala Ser Ser Thr Asn Pro Asn Ala Thr Asn Gly Gly Ser
           195                 200                 205
   Asp Phe Gly Arg Ile Asn Val Ala Asn Gly Ile Lys Leu Asp Ser Gly
       210                 215                 220
   Ser Glu Asn Val Thr Leu Thr His Cys Lys Asp Lys Val Cys Asp Arg
   225                 230                 235                 240
   Asp Phe Leu Asp Glu Glu Ala Pro Pro Lys Ser Glu Phe Glu Lys Leu
                   245                 250                 255
   Ser Asp Glu Glu Lys Ile Asn Lys Tyr Lys Lys Asp Glu Gln Arg Glu
               260                 265                 270
   Asn Phe Val Gly Leu Val Ala Asp Arg Val Glu Lys Asn Gly Thr Asn
           275                 280                 285
   Lys Tyr Val Ile Ile Tyr Lys Asp Lys Ser Ala Ser Ser Ser Ser Ala
       290                 295                 300
   Arg Phe Arg Arg Ser Ala Arg Ser Arg Arg Ser Leu Pro Ala Glu Met
   305                 310                 315                 320
   Pro Leu Ile Pro Val Asn Gln Ala Asp Thr Leu Ile Val Asp Gly Glu
                   325                 330                 335
   Ala Val Ser Leu Thr Gly His Ser Gly Asn Ile Phe Ala Pro Glu Gly
               340                 345                 350
   Asn Tyr Arg Tyr Leu Thr Tyr Gly Ala Glu Lys Leu Ser Gly Gly Ser
           355                 360                 365
   Tyr Ala Leu Ser Val Gln Gly Glu Pro Ala Lys Gly Glu Met Leu Ala
       370                 375                 380
   Gly Thr Ala Val Tyr Asn Gly Glu Val Leu His Phe His Met Glu Asn
   385                 390                 395                 400
   Gly Arg Pro Ser Pro Ser Gly Gly Arg Phe Ala Ala Lys Val Asp Phe
                   405                 410                 415
   Gly Ser Lys Ser Val Asp Gly Ile Ile Asp Ser Gly Asp Asp Leu His
               420                 425                 430
   Met Gly Thr Gln Lys Phe Lys Ala Val Ile Asp Gly Asn Gly Phe Lys
           435                 440                 445
   Gly Thr Trp Thr Glu Asn Gly Gly Gly Asp Val Ser Gly Arg Phe Tyr
       450                 455                 460
   Gly Pro Ala Gly Glu Glu Val Ala Gly Lys Tyr Ser Tyr Arg Pro Thr
   465                 470                 475                 480
   Asp Ala Glu Lys Gly Gly Phe Gly Val Phe Ala Gly Lys Lys Glu Gln
   485 490 495
   Asp
<210> 26
   <211> 364
   <212> PRT
   <213> Neisseria meningitidis
<400> 26
   Met Ser Met Lys His Phe Pro Ser Lys Val Leu Thr Thr Ala Ile Leu
   1               5                   10                  15
   Ala Thr Phe Cys Ser Gly Ala Leu Ala Ala Thr Ser Asp Asp Asp Val
               20                  25                  30
   Lys Lys Ala Ala Thr Val Ala Ile Val Ala Ala Tyr Asn Asn Gly Gln
           35                  40                  45
   Glu Ile Asn Gly Phe Lys Ala Gly Glu Thr Ile Tyr Asp Ile Gly Glu
       50                  55                  60
   Asp Gly Thr Ile Thr Gln Lys Asp Ala Thr Ala Ala Asp Val Glu Ala
   65                  70                  75                  80
   Asp Asp Phe Lys Gly Leu Gly Leu Lys Lys Val Val Thr Asn Leu Thr
                   85                  90                  95
   Lys Thr Val Asn Glu Asn Lys Gln Asn Val Asp Ala Lys Val Lys Ala
               100                 105                 110
   Ala Glu Ser Glu Ile Glu Lys Leu Thr Thr Lys Leu Ala Asp Thr Asp
           115                 120                 125
   Ala Ala Leu Ala Asp Thr Asp Ala Ala Leu Asp Glu Thr Thr Asn Ala
       130                 135                 140
   Leu Asn Lys Leu Gly Glu Asn Ile Thr Thr Phe Ala Glu Glu Thr Lys
   145                 150                 155                 160
   Thr Asn Ile Val Lys Ile Asp Glu Lys Leu Glu Ala Val Ala Asp Thr
                   165                 170                 175
   Val Asp Lys His Ala Glu Ala Phe Asn Asp Ile Ala Asp Ser Leu Asp
               180                 185                 190
   Glu Thr Asn Thr Lys Ala Asp Glu Ala Val Lys Thr Ala Asn Glu Ala
           195                 200                 205
   Lys Gln Thr Ala Glu Glu Thr Lys Gln Asn Val Asp Ala Lys Val Lys
       210                 215                 220
   Ala Ala Glu Thr Ala Ala Gly Lys Ala Glu Ala Ala Ala Gly Thr Ala
   225                 230                 235                 240
   Asn Thr Ala Ala Asp Lys Ala Glu Ala Val Ala Ala Lys Val Thr Asp
                   245                 250                 255
   Ile Lys Ala Asp Ile Ala Thr Asn Lys Ala Asp Ile Ala Lys Asn Ser
               260                 265                 270
   Ala Arg Ile Asp Ser Leu Asp Lys Asn Val Ala Asn Leu Arg Lys Glu
           275                 280                 285
   Thr Arg Gln Gly Leu Ala Glu Gln Ala Ala Leu Ser Gly Leu Phe Gln
       290                 295                 300
   Pro Tyr Asn Val Gly Arg Phe Asn Val Thr Ala Ala Val Gly Gly Tyr
   305                 310                 315                 320
   Lys Ser Glu Ser Ala Val Ala Ile Gly Thr Gly Phe Arg Phe Thr Glu
                   325                 330                 335
   Asn Phe Ala Ala Lys Ala Gly Val Ala Val Gly Thr Ser Ser Gly Ser
               340                 345                 350
   Ser Ala Ala Tyr His Val Gly Val Asn Tyr Glu Trp
           355                 360
<210> 27
   <211> 396
   <212> PRT
   <213> Artificial Sequence
<220>
   <223> Fusion protein PilE-fHBP
<400> 27
   Ser Ala Val Thr Glu Tyr Tyr Leu Asn His Gly Glu Trp Pro Gly Asp
   1               5                   10                  15
   Asn Ser Ser Ala Gly Val Ala Thr Ser Ala Asp Ile Lys Gly Lys Tyr
               20                  25                  30
   Val Gln Ser Val Thr Val Ala Asn Gly Val Ile Thr Ala Gln Met Ala
           35                  40                  45
   Ser Ser Asn Val Asn Asn Glu Ile Lys Ser Lys Lys Leu Ser Leu Trp
       50                  55                  60
   Ala Lys Arg Gln Asn Gly Ser Val Lys Trp Phe Cys Gly Gln Pro Val
   65                  70                  75                  80
   Thr Arg Thr Thr Ala Thr Ala Thr Asp Val Ala Ala Ala Asn Gly Lys
                   85                  90                  95
   Thr Asp Asp Lys Ile Asn Thr Lys His Leu Pro Ser Thr Cys Arg Asp
               100                 105                 110
   Asp Ser Ser Ala Ser Met Thr Arg Ser Lys Pro Val Asn Arg Thr Ala
           115                 120                 125
   Phe Cys Cys Leu Ser Leu Thr Ala Ala Leu Ile Leu Thr Ala Cys Ser
       130                 135                 140
   Ser Gly Gly Gly Gly Val Ala Ala Asp Ile Gly Ala Gly Leu Ala Asp
   145                 150                 155                 160
   Ala Leu Thr Ala Pro Leu Asp His Lys Asp Lys Ser Leu Gln Ser Leu
                   165                 170                 175
   Thr Leu Asp Gln Ser Val Arg Lys Asn Glu Lys Leu Lys Leu Ala Ala
               180                 185                 190
   Gln Gly Ala Glu Lys Thr Tyr Gly Asn Gly Asp Ser Leu Asn Thr Gly
           195                 200                 205
   Lys Leu Lys Asn Asp Lys Val Ser Arg Phe Asp Phe Ile Arg Gln Ile
       210                 215                 220
   Glu Val Asp Gly Gln Leu Ile Thr Leu Glu Ser Gly Glu Phe Gln Ile
   225                 230                 235                 240
   Tyr Lys Gln Asp His Ser Ala Val Val Ala Leu Gln Ile Glu Lys Ile
                   245                 250                 255
   Asn Asn Pro Asp Lys Ile Asp Ser Leu Ile Asn Gln Arg Ser Phe Leu
               260                 265                 270
   Val Ser Gly Leu Gly Gly Glu His Thr Ala Phe Asn Gln Leu Pro Gly
           275                 280                 285
   Gly Lys Ala Glu Tyr His Gly Lys Ala Phe Ser Ser Asp Asp Ala Gly
       290                 295                 300
   Gly Lys Leu Thr Tyr Thr Ile Asp Phe Ala Ala Lys Gln Gly His Gly
   305                 310                 315                 320
   Lys Ile Glu His Leu Lys Thr Pro Glu Gln Asn Val Glu Leu Ala Ala
                   325                 330                 335
   Ala Glu Leu Lys Ala Asp Glu Lys Ser His Ala Val Ile Leu Gly Asp
               340                 345                 350
   Thr Arg Tyr Gly Ser Glu Glu Lys Gly Thr Tyr His Leu Ala Leu Phe
           355                 360                 365
   Gly Asp Arg Ala Gln Glu Ile Ala Gly Ser Ala Thr Val Lys Ile Gly
       370                 375                 380
   Glu Lys Val His Glu Ile Gly Ile Ala Gly Lys Gln
   385                 390                 395
<210> 28
   <211> 314
   <212> PRT
   <213> Artificial Sequence
<220>
   <223> Fusion protein PilE-fHBP
<400> 28
   Cys Gly Gln Pro Val Thr Arg Thr Thr Ala Thr Ala Thr Asp Val Ala
   1               5                   10                  15
   Ala Ala Asn Gly Lys Thr Asp Asp Lys Ile Asn Thr Lys His Leu Pro
               20                  25                  30
   Ser Thr Cys Met Thr Arg Ser Lys Pro Val Asn Arg Thr Ala Phe Cys
           35                  40                  45
   Cys Leu Ser Leu Thr Ala Ala Leu Ile Leu Thr Ala Cys Ser Ser Gly
       50                  55                  60
   Gly Gly Gly Val Ala Ala Asp Ile Gly Ala Gly Leu Ala Asp Ala Leu
   65                  70                  75                  80
   Thr Ala Pro Leu Asp His Lys Asp Lys Ser Leu Gln Ser Leu Thr Leu
                   85                  90                  95
   Asp Gln Ser Val Arg Lys Asn Glu Lys Leu Lys Leu Ala Ala Gln Gly
               100                 105                 110
   Ala Glu Lys Thr Tyr Gly Asn Gly Asp Ser Leu Asn Thr Gly Lys Leu
           115                 120                 125
   Lys Asn Asp Lys Val Ser Arg Phe Asp Phe Ile Arg Gln Ile Glu Val
       130                 135                 140
   Asp Gly Gln Leu Ile Thr Leu Glu Ser Gly Glu Phe Gln Ile Tyr Lys
   145 150 155 160
   Gln Asp His Ser Ala Val Val Ala Leu Gln Ile Glu Lys Ile Asn Asn
   165 170 175
   Pro Asp Lys Ile Asp Ser Leu Ile Asn Gln Arg Ser Phe Leu Val Ser
   180 185 190
   Gly Leu Gly Gly Glu His Thr Ala Phe Asn Gln Leu Pro Gly Gly Lys
   195 200 205
   Ala Glu Tyr His Gly Lys Ala Phe Ser Ser Asp Asp Ala Gly Gly Lys
   210 215 220
   Leu Thr Tyr Thr Ile Asp Phe Ala Ala Lys Gln Gly His Gly Lys Ile
   225 230 235 240
   Glu His Leu Lys Thr Pro Glu Gln Asn Val Glu Leu Ala Ala Ala Glu
   245 250 255
   Leu Lys Ala Asp Glu Lys Ser His Ala Val Ile Leu Gly Asp Thr Arg
   260 265 270
   Tyr Gly Ser Glu Glu Lys Gly Thr Tyr His Leu Ala Leu Phe Gly Asp
   275 280 285
   Arg Ala Gln Glu Ile Ala Gly Ser Ala Thr Val Lys Ile Gly Glu Lys
   290 295 300
   Val His Glu Ile Gly Ile Ala Gly Lys Gln
   305 310
<210> 29
   <211> 355
   <212> PRT
   <213> Artificial Sequence
<220>
   <223> Fusion protein PilV-fHBP
<400> 29
   Thr Met Glu Arg Tyr Tyr Arg Gln Lys Gly Thr Phe Lys Thr Tyr Asp
   1               5                   10                  15
   Lys Asn Lys Leu Lys Gln Asn Lys Tyr Phe Asn Val Thr Leu Ser Lys
               20                  25                  30
   Val Ser Pro Asp His Phe Thr Leu Gln Ala Asp Pro Asn Pro Thr Thr
               35                  40                  45
   Asn Asp Gly Glu Thr Cys Val Val Thr Leu Asp Gly Gly Thr Ile Ala
       50                  55                  60
   Ala Ser Gly Thr Asn Gln Ser Cys Pro Gly Phe Asp Met Thr Arg Ser
   65                  70                  75                  80
   Lys Pro Val Asn Arg Thr Ala Phe Cys Cys Leu Ser Leu Thr Ala Ala
                   85                  90                  95
   Leu Ile Leu Thr Ala Cys Ser Ser Gly Gly Gly Gly Val Ala Ala Asp
               100                 105                 110
   Ile Gly Ala Gly Leu Ala Asp Ala Leu Thr Ala Pro Leu Asp His Lys
           115                 120                 125
   Asp Lys Ser Leu Gln Ser Leu Thr Leu Asp Gln Ser Val Arg Lys Asn
       130                 135                 140
   Glu Lys Leu Lys Leu Ala Ala Gln Gly Ala Glu Lys Thr Tyr Gly Asn
   145                 150                 155                 160
   Gly Asp Ser Leu Asn Thr Gly Lys Leu Lys Asn Asp Lys Val Ser Arg
                   165                 170                 175
   Phe Asp Phe Ile Arg Gln Ile Glu Val Asp Gly Gln Leu Ile Thr Leu
               180                 185                 190
   Glu Ser Gly Glu Phe Gln Ile Tyr Lys Gln Asp His Ser Ala Val Val
           195                 200                 205
   Ala Leu Gln Ile Glu Lys Ile Asn Asn Pro Asp Lys Ile Asp Ser Leu
       210                 215                 220
   Ile Asn Gln Arg Ser Phe Leu Val Ser Gly Leu Gly Gly Glu His Thr
   225                 230                 235                 240
   Ala Phe Asn Gln Leu Pro Gly Gly Lys Ala Glu Tyr His Gly Lys Ala
                   245                 250                 255
   Phe Ser Ser Asp Asp Ala Gly Gly Lys Leu Thr Tyr Thr Ile Asp Phe
               260                 265                 270
   Ala Ala Lys Gln Gly His Gly Lys Ile Glu His Leu Lys Thr Pro Glu
           275                 280                 285
   Gln Asn Val Glu Leu Ala Ala Ala Glu Leu Lys Ala Asp Glu Lys Ser
       290                 295                 300
   His Ala Val Ile Leu Gly Asp Thr Arg Tyr Gly Ser Glu Glu Lys Gly
   305                 310                 315                 320
   Thr Tyr His Leu Ala Leu Phe Gly Asp Arg Ala Gln Glu Ile Ala Gly
                   325                 330                 335
   Ser Ala Thr Val Lys Ile Gly Glu Lys Val His Glu Ile Gly Ile Ala
               340                 345                 350
   Gly Lys Gln
           355
<210> 30
   <211> 306
   <212> PRT
   <213> Artificial Sequence
<220>
   <223> Fusion protein PilV-fHBP
<400> 30
   Gly Glu Thr Cys Val Val Thr Leu Asn Asp Gly Gly Thr Ile Ala Ala
   1               5                   10                  15
   Ser Gly Thr Asn Gln Ser Cys Pro Gly Phe Asp Met Thr Arg Ser Lys
               20                  25                  30
   Pro Val Asn Arg Thr Ala Phe Cys Cys Leu Ser Leu Thr Ala Ala Leu
           35                  40                  45
   Ile Leu Thr Ala Cys Ser Ser Gly Gly Gly Gly Val Ala Ala Asp Ile
       50                  55                  60
   Gly Ala Gly Leu Ala Asp Ala Leu Thr Ala Pro Leu Asp His Lys Asp
   65                  70                  75                  80
   Lys Ser Leu Gln Ser Leu Thr Leu Asp Gln Ser Val Arg Lys Asn Glu
                   85                  90                  95
   Lys Leu Lys Leu Ala Ala Gln Gly Ala Glu Lys Thr Tyr Gly Asn Gly
               100                 105                 110
   Asp Ser Leu Asn Thr Gly Lys Leu Lys Asn Asp Lys Val Ser Arg Phe
           115                 120                 125
   Asp Phe Ile Arg Gln Ile Glu Val Asp Gly Gln Leu Ile Thr Leu Glu
       130                 135                 140
   Ser Gly Glu Phe Gln Ile Tyr Lys Gln Asp His Ser Ala Val Val Ala
   145                 150                 155                 160
   Leu Gln Ile Glu Lys Ile Asn Asn Pro Asp Lys Ile Asp Ser Leu Ile
                   165                 170                 175
   Asn Gln Arg Ser Phe Leu Val Ser Gly Leu Gly Gly Glu His Thr Ala
               180                 185                 190
   Phe Asn Gln Leu Pro Gly Gly Lys Ala Glu Tyr His Gly Lys Ala Phe
           195                 200                 205
   Ser Ser Asp Asp Ala Gly Gly Lys Leu Thr Tyr Thr Ile Asp Phe Ala
       210                 215                 220
   Ala Lys Gln Gly His Gly Lys Ile Glu His Leu Lys Thr Pro Glu Gln
   225                 230                 235                 240
   Asn Val Glu Leu Ala Ala Ala Glu Leu Lys Ala Asp Glu Lys Ser His
                   245                 250                 255
   Ala Val Ile Leu Gly Asp Thr Arg Tyr Gly Ser Glu Glu Lys Gly Thr
               260                 265                 270
   Tyr His Leu Ala Leu Phe Gly Asp Arg Ala Gln Glu Ile Ala Gly Ser
           275                 280                 285
   Ala Thr Val Lys Ile Gly Glu Lys Val His Glu Ile Gly Ile Ala Gly
       290                 295                 300
   Lys Gln
   305

## Description

The present invention stems from the finding that the interaction between the β2 adrenoceptor (β2AR) and type IV pilus-associated proteins initiates a process leading to the opening of the blood-brain barrier. The invention therefore pertains to a vaccine for preventing the spreading of meningococci into the meningeal space, wherein said vaccine allows the production of antibodies inhibiting the interaction between the type IV pilus-associated proteins and the β2AR.

Meningococcus (*N. meningitidis*, *Nm*), a cause of epidemic meningitis and sepsis, is a commensal Gram-negative bacterium of the human nasopharynx. After bloodstream invasion, virulent encapsulated bacteria adhere to brain endothelial cells and proliferate onto the apical surface of host cells to form microcolonies at the site of initial bacterial attachment, then cross the Blood Brain Barrier (BBB) to colonize meninges (Nassif et al., 2002, Trends Microbiol 10, 227-232).

Both bacterial adhesion and subsequent *Nm*-promoted signaling in host cells depend on the presence of bacterial filamentous structures, the type IV pili (tfp) (Nassif et al., 1994, Proc Natl Acad Sci U S A 91, 3769-3773; Virji et al., 1991, Mol Microbiol 5, 1831-1841) and of cellular receptor(s), which still remain elusive.

*In vitro* experiments under controlled flow indicated that meningococcal adhesion could only occur at low shear stress levels, which are observed in capillaries upon transient reduction in flow. After initial attachment, bacteria acquire the capacity to resist higher blood velocities and to multiply, forming colonies. Cellular and molecular mechanisms of this tighter adhesion are initiated by tfp-dependent activation of host cell signaling cascades. *In vitro,* encapsulated meningococci elicit the organization of specific "honeycomb"-shaped molecular complexes underneath bacterial colonies, referred to as "cortical plaques". Cortical plaques result from the recruitment of molecular linkers, such as ezrin and moesin, adhesion molecules and membrane receptors, and from localized polymerization of cortical actin, leading to the formation of microvilli-like plasma membrane protrusions among bacteria, which are crucial for *Nm* colonies to resist shear stress and colonize blood vessels. Changes of the host cell cytoskeleton induced by *Nm* are associated with the turning on of multiple signaling events including the activation of the Src kinase, the activation of the GTPases Rho, Cdc42, and Rac, the PI3-kinase-mediated recruitment of cortactin and the activation of mitogen-activated kinases.

Whether *Nm* ultimately pass through the barrier of brain endothelial cells by transcytosis (Nassif et al., 2002, Trends Microbiol 10, 227-232) or through cell junctions has been debated for a long time. Recently, however, *Nm* were shown to recruit endothelial cell adhesion molecules, such as VE-cadherin, into cortical plaques. This recruitment leads to the formation of ectopic intercellular junctional domains at the site of bacteria-host cell interaction, depletion of junctional proteins at the cell-cell interface, opening of intercellular junctions and subsequent crossing of the BBB (Coureuil et al., 2009, Science 325, 83-87).

Pleiotropic signaling events elicited by *Nm* in endothelial cells, which allow bacteria to resist to blood flow and cross the BBB, might either proceed from independent parallel signaling pathways, or, more likely, depend on a multimolecular signaling complex scaffolded downstream of an single host cell receptor. Among candidate proteins capable of scaffolding multiple cellular signals, β-arrestins (referred here to βarr1 and βarr2), two ubiquitously expressed molecules initially described as regulators of G protein coupled receptors (GPCRs), participate in many of the signaling pathways that are elicited by meningococci. For example, βarrs operate as adaptor proteins to recruit and activate Src under agonist-occupied receptors. In addition, βarrs act as scaffolds for GPCR-mediated activation of the MAPKs ERK1/2 into cytoplasmic compartments, and drive localized activation of cortical actin polymerization. βarrs were also shown to contribute to the activation of several Ras-family GTPases. Finally, the junctional depletion of adhesion molecules with subsequent enhanced vascular permeability, promoted by activation of the vascular endothelial growth factor (VEGF), involves βarr-dependent endocytosis of phospho-VE-cadherin into clathrin-coated vesicles.

However, the cellular receptor which allows internalization of *N. meningitis* by opening the intercellular junctions or initiating transcytosis still remains unknown. The tfp proteins involved in the interaction with that cellular receptor are also unknown.

Crossing of the blood-brain barrier (BBB) is a burning problem in the field of medicine. Indeed, the major problem encountered when wanting to treat CNS disorders is how to deliver a therapeutic agent into the brain.

It has been shown that a direct, topical application of a β-adrenoceptor agonist to the brain paremchyma increased the permeability of the BBB (Chi et al. 1998, Neurol Res. 20:259-64). However, Chi *et al.* do not teach whether increased BBB permeability is a direct or an indirect effect of β-adrenoceptor activation, and fail to elucidate which β-adrenoceptors are involved in the observed increase of BBB permeability. In addition, Chi *et al.* neither teach nor suggest any mean allowing a systemic administration of a therapeutic agent.

The main clinical feature of meningococcal infection is meningitis. Paradoxically, *Neisseria meningitidis* is a frequent asymptomatic colonizer of the human nasopharynx, and only a very small proportion of infections proceed to a sustained bacteraemia. In the majority of bacteremic patients the presence of *N.meningitdis* in the bloodstream will remain asymptomatic. However during the blood phase, the bacteria will interact with the brain endothelial cells and invade the meninges to be responsible for meningitis which is the first clinical complication of meningococcal dissemination in 70% of patients. In 30% of bacteremic patients the presence of *meningococci* in the bloodstream will lead to clinical symptoms of septicemia with in some cases a purpura fulminans. In the latter case the bacteria cross the blood brain barrier; however, the meningitis symptoms are not at the forefront of the clinical presentation.

The reasons why meningococcal disseminate from the nasopharynx into the bloodstream in some individuals and not in others remain unclear, but human genetic polymorphism is likely to be important in determining the outcome of nasopharyngeal colonization. In addition, all *meningococci* do not have the same pathogenic potential. Indeed, analysis of results from multilocus sequence typing (MLST) have demonstrated the existence of distinct phylogenetic groups (clonal complexes), some of which are more likely to be isolated from patients than others. These are the so-called "hyper-virulent" or "hyperinvasive" lineages. Recently, the presence of a prophage has been shown to be responsible for a large proportion of invasiveness of strains belonging to hyperinvasive lineages. This element inserted into the chromosome can be induced to produce a filamentous phage.

Few bacterial pathogens have the ability to cross the blood brain barrier and to invade the meninges. After the neonatal period those pathogens are, beside *Neisseria meningitdis*, mostly *Streptocccus pneumoniae*, *Haemophilus influenzae*. During the neonatal period *Streptococcus agalactiae* and *Escherichia coli* are routinely isolated. Finally, at all ages but rarely and in some circumstances *Mycobacterium tuberculosis* and *Listeria* monocytogenes are responsible for meningitis. However among those pathogens the one that achieves this goal the most constantly and the most efficiently is *Neisseria meningitidis*, thus pointing out the efficiency with which this pathogen can cross the blood brain barrier. A study using postmortem examination has shown that meningeal invasion is a consequence of the direct interaction of *N.meningitidis* with the brain endothelial cells (Pron et al, Journal of Infectious Diseases, 1997, 176(5):1285-92). In vitro experiments using a brain endothelial cell line has shown that the type IV pili of *N.meningitidis* are responsible for the formation of microcolonies on the apical surface of endothelial cell capillaries. The formation of these microcolonies leads to a signalling to the endothelial cells and the recruitment of junctional molecules at the site of the bacterial cell interaction, thus depleting the intercellular junctions and opening the paracellular route. *N.meningitidis* can then cross this monolayer between two adjacent cells.

A vaccine aiming at protecting against cerebrospinal meningitis will be best if it prevents bacterial dissemination in the bloodstream by inducing bactericidal antibodies and inhibits the ability of the bacteria to cross the blood brain barrier. Most of the current strategies aiming at engineering a vaccine against meningococcal infection concentrate solely on the production of bactericidal antibodies. Considering that the dissemination of the bacteria into the blood remains asymptomatic until it crosses the blood brain barrier, preventing the ability of the bacteria to signal to brain endothelial cells will prevent meningeal dissemination and bacterial meningitis. The inclusion in a meningococcal vaccine of epitopes inducing antibodies preventing meningococcal crossing of the blood brain barrier will therefore be beneficial for the prevention of cerebrospinal meningitis.

Thus there is a need for vaccines preventing the meningococci to cross the blood brain barrier and consequently preventing cerebrospinal meningitis.

Cehovin et al. ("Sequence conservation of pilus subunits in Neisseiria meningitidis", VACCINE, vol.28, no.30, 5 July 2010, pages 4817-4826) reports sequence conservation in pilus subunits in *Neisseria meningitidis* and implications thereof for the reconsideration of pilus subunits as vaccine antigens. It discloses that, contrary to what was previously accepted, a vaccine based on PILE would be a possible approach, and suggests a vaccine comprising PILV, PILX and comP.

Orihuela et al. ("Laminin receptor initiates bacterial contact with the blood brain barrier in experimental meningitis model", JOURNAL OF CLINICAL INVESTIGATION, vol.119, no.6, June 2009, pages 1638-1646) discloses that the laminin receptor on vascular endothelial cells interacts with certain bacterial adhesins such as pilQ protein of *Neisseria meningitidis.*

The inventors have surprinsingly found that the signalling leading to the opening of the blood brain barrier is a consequence of the interaction of two pilus components, the major pilin PilE and the minor pilin PilV, with the N-terminal portion of the beta2 adrenergic receptor. This interaction leads to the activation of the beta arrestin and the subsequent signalling into the host cells followed by the opening of the intercellular junction proteins.

Thus the inventors have found that a vaccine inducing antibodies inhibiting the interaction between the components of the type IV pili carrying the recognition domain to the beta2 adrenergic receptor responsible for meningococcal signalling, *i.e.* these components are the major pilin PilE and the minor pilin PilV, is particularly useful for preventing cerebrospinal meningitis. More particularly, they have discovered that purified PilE and PilV or portion of these peptides, used either in association, or fused in the same polypeptide chain to increase their immunogenicity, are the best candidate to induce the production of protective antibodies, as long as the fragment of each molecule encompass the domain of interaction with the beta2 adrenergic receptor.

The mature protein of PilE is approximately 145-160 residues long after processing by a prepilin peptidase. It has a conserved hydrophobic N-terminal 25 residue and a carboxy-terminal disulphide bond. The conserved hydrophobic N-terminal moiety that is embedded in the core of the pilus is involved in the cohesion of the subunits as a fibre. The polymorphic C-terminus of pilin is partially exposed at the surface of the assembled pilus. The pilin also harbours two cysteine residues that build a disulfide bridge, involved in the formation of the surface accessible, variable and immune dominant part of the pilus. Pilins are therefore packed through internal hydrophobic interactions between conserved N-terminal α helices, leaving hypervariable C-terminal globular regions exposed. The major pilin is encoded by the PilE gene. The fragment contained in the vaccine will therefore be either a complete pilin or a fragment of the molecule expressing the epitope binding to the N-terminal portion of the beta2 adrenergic receptor. In addition considering the great variability of pilin, different variants may be included in the vaccine. However, it is likely that the domain binding to the beta2 adrenergic receptor has a conserved structure between strains.

The minor pilin PilV is structurally very similar to the major pilin but these genes are present in every strain tested and are remarkably conserved between different serogroups and clonal complexes. As for the major pilin PilE, a fragment deleted of the N-terminal hydrophobic portion or even a smaller fragment can be used as long as antibodies against this fragment are capable of inducing antibodies inhibiting the endothelial cell signaling.

### DESCRIPTION OF THE INVENTION

The inventors have surprisingly found that *Nm* colonies at the cell surface of a human brain endothelial cell line promote the translocation of βarrs to the inner surface of plasma membrane, facing bacteria. βarrs translocated under the colonies serve as a scaffolding platform for signaling events elicited by *Nm.* Among the GPCRs expressed in the cell line, only the β2-adrenoceptor (β2AR) plays a permissive role in the formation of cortical plaques under colonies and in bacterial crossing of cell monolayers. These observations reveal the requisition of a β2-adrenergic/β-arrestin signaling pathway by *Nm* to promote stable adhesion onto human brain endothelial cells and subsequent crossing of the BBB.

More specifically, it has been shown that interaction of β2AR, in particular of the N-terminus of β2AR, with type IV pilus-associated proteins such as PilE or PilV, initiates the process allowing *N. meningitidis* to open and to traverse the BBB.

The inventors have surprisingly found that a vaccine inducing antibodies inhibiting the interaction between the components of the type IV pili carrying the recognition domain to the beta2 adrenergic receptor responsible for meningococcal signalling, *i.e.* major pilin PilE and minor pilin PilV, is particularly useful for preventing cerebrospinal meningitis.

The present invention is as defined in the subject matter of claims 1-6 i.e. relates to:
1. A protein that comprises or consists of:
   (a) the meningococcal PilV sequence SEQ ID NO: 2 or a sequence at least 80% identical to SEQ ID NO:2, or
   (b) an immunogenic fragment of (a) that comprises or consists of SEQ ID N°21, or
   (c) an immunogenic fragment of (a) that comprises or consists of a sequence that is at least 80% identical to SEQ ID NO: 21, or
   (d) an immunogenic fragment of (a) that comprises or consists of SEQ ID N°19, or
   (e) an immunogenic fragment of (a) that comprises or consists of a sequence that is at least 80% identical to SEQ ID N°19;
   for use as a vaccine for preventing the spreading of meningococci into the meningeal space, wherein said vaccine induces the production of protective antibodies inhibiting the interaction between PilV and mammalian β2 adrenoreceptor.
2. A combination of at least two of:
   a) a protein that comprises or consists of :
      (i) the meningococcal PilV sequence SEQ ID NO: 2 or a sequence at least 80% identical to SEQ ID NO:2, or
      (ii) an immunogenic fragment of (i) that comprises or consists of SEQ ID N°21, or
      (iii) an immunogenic fragment of (i) that comprises or consists of a sequence that is at least 80% identical to SEQ ID NO: 21, or
      (iv) an immunogenic fragment of (i) that comprises or consists of SEQ ID N°19, or
      (v) an immunogenic fragment of (i) that comprises or consists of a sequence that is at least 80% identical to SEQ ID N°19; and
   b) a meningococcal vaccine antigen;
   for simultaneous or sequential use as a vaccine for preventing the spreading of meningococci into the meningeal space, wherein said vaccine induces the production of protective antibodies inhibiting the interaction between PilV and mammalian β2 adrenoreceptor, wherein said meningococcal vaccine antigen is not a PilE, PilV, or an immunogenic fragment thereof.
3. A fusion protein comprising at least two of:
   a) a protein that comprises or consists of :
      (i) the meningococcal PilV sequence SEQ ID NO: 2 or a sequence at least 80% identical to SEQ ID NO:2, or
      (ii) an immunogenic fragment of (i) that comprises or consists of SEQ ID N°21, or
      (iii) an immunogenic fragment of (i) that comprises or consists of a sequence that is at least 80% identical to SEQ ID NO: 21, or
      (iv) an immunogenic fragment of (i) that comprises or consists of SEQ ID N°19, or
      (v) an immunogenic fragment of (i) that comprises or consists of a sequence that is at least 80% identical to SEQ ID N°19; and
   b) a meningococcal vaccine antigen,
   wherein said meningococcal vaccine antigen is not PilE, PilV, or an immunogenic fragment thereof.
4. A fusion protein according to claim 3 for use as a vaccine for preventing the spreading of meningococci into the meningeal space, wherein said vaccine induces the production of protective antibodies inhibiting the interaction between PilV and mammalian β2 adrenoreceptor, wherein said meningococcal vaccine antigen is not PilE, PilV, or an immunogenic fragment thereof.
5. A vaccine composition or an immunogenic composition for use for preventing the spreading of meningococci into the meningeal space by the production of protective antibodies inhibiting the interaction between PilV and mammalian β2 adrenoreceptor, wherein said vaccine or immunogenic composition comprises or consists of:
   - a protein that comprises or consists of:
      (a) the meningococcal PilV sequence SEQ ID NO: 2 or a sequence at least 80% identical to SEQ ID NO:2, or
      (b) an immunogenic fragment of (a) that comprises or consists of SEQ ID N°21, or
      (c) an immunogenic fragment of (a) that comprises or consists of a sequence that is at least 80% identical to SEQ ID NO: 21, or
      (d) an immunogenic fragment of (a) that comprises or consists of SEQ ID N°19, or
      (e) an immunogenic fragment of (a) that comprises or consists of a sequence that is at least 80% identical to SEQ ID N°19;
   - a combination of at least two of:
      a) a protein that comprises or consists of :
         (i) the meningococcal PilV sequence SEQ ID NO: 2 or a sequence at least 80% identical to SEQ ID NO:2, or
         (ii) an immunogenic fragment of (i) that comprises or consists of SEQ ID N°21, or
         (iii) an immunogenic fragment of (i) that comprises or consists of a sequence that is at least 80% identical to SEQ ID NO: 21, or
         (iv) an immunogenic fragment of (i) that comprises or consists of SEQ ID N°19, or
         (v) an immunogenic fragment of (i) that comprises or consists of a sequence that is at least 80% identical to SEQ ID N°19; and
      b) a meningococcal vaccine antigen; or
   - a fusion protein according to claim 3.
6. A protein for the use according to claim 1, a combination for the use according to claim 2, a fusion protein according to claim 3, a fusion protein for the use according to claim 4 or a vaccine or an immunogenic composition for the use according to claim 5, wherein said immunogenic fragment does not carry the hydrophobic domain of sequence SEQ ID NO: 17.

### Definitions

By "PilE protein" and "PilV proteins", it is meant PilE and PilV proteins derived from any meningococcal serogroup, e.g. from a meningococcus of serogroup A, B, C, Y or W135. Preferably, said PilE and PilV proteins comprise or consist of:
a) the sequence of SEQ ID NO: 1 (*N. meningitidis* PilE);
b) the sequence of SEQ ID NO: 2 (*N. meningitidis* PilV); or
c) a sequence that is at least 80, 85, 90, 95 or 99% identical to the sequence of (a) or (b).

By a polypeptide having an amino acid sequence at least, for example, 95% "identical" to a query amino acid sequence of the present invention, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% (5 of 100) of the amino acid residues in the subject sequence may be inserted, deleted, or substituted with another amino acid.

In the frame of the present application, the percentage of identity is calculated using a global alignment (*i.e.* the two sequences are compared over their entire length). Methods for comparing the identity of two or more sequences are well known in the art. The « needle » program, which uses the Needleman-Wunsch global alignment algorithm (Needleman and Wunsch, 1970 J. Mol. Biol. 48:443-453) to find the optimum alignment (including gaps) of two sequences when considering their entire length, may for example be used. The needle program is for example available on the ebi.ac.uk world wide web site. The percentage of identity in accordance with the invention is preferably calculated using the EMBOSS::needle (global) program with a "Gap Open" parameter equal to 10.0, a "Gap Extend" parameter equal to 0.5, and a Blosum62 matrix.

Proteins consisting of an amino acid sequence "at least 80%, 85%, 90%, 95% or 99% identical" to a reference sequence may comprise mutations such as deletions, insertions and/or substitutions compared to the reference sequence. In case of substitutions, the protein consisting of an amino acid sequence at least 80%, 85%, 90%, 95% or 99% identical to a reference sequence may correspond to a homologous sequence derived from another species than the reference sequence. In another preferred embodiment, the substitution preferably corresponds to a conservative substitution as indicated in the table below.

| **Conservative substitutions** | **Type of Amino Acid** |
|---|---|
| Ala, Val, Leu, Ile, Met, Pro, Phe, Trp | Amino acids with aliphatic hydrophobic side chains |
| Ser, Tyr, Asn, Gln, Cys | Amino acids with uncharged but polar side chains |
| Asp, Glu | Amino acids with acidic side chains |
| Lys, Arg, His | Amino acids with basic side chains |
| Gly | Neutral side chain |

Mammalian β2 adrenoceptors (abbreviated β2ARs) are well known to the skilled in the art. For example, the human β2 adrenoceptor encompasses the protein having the sequence of SEQ ID NO: 3 as well as allelic variants and splice variants thereof. In the frame of the present invention, the mammalian β2AR may for example correspond to a human, a rat, a mouse, a guinea pig, a Chinese hamster, a rabbit or a monkey β2AR.

By "protective antibodies", it is meant antibodies inhibiting the interaction between PilV and β2AR. More particularly, said antibodies inhibit the interaction between the N-terminus of β2AR and, PilV. Thus, said protective antibodies may for example recognize a linear epitope either comprised within the recognition domain to the β2AR of PilV, or comprising at least 1, 2, 3, 4, 5, 6, 7, 8, 9 or 10 consecutive amino acids located within the recognition domain to the β2AR of PilV. Alternatively, the antibody may bind to a conformational epitope comprising at least 1, 2, 3, 4, 5, 6, 7, 8, 9 or 10 amino acids located within the recognition domain to the β2AR of PilV.

By "immunogenic fragment" is meant a contiguous portion of PilE or PilV which has the same or substantially the same immunogenic activity as PilE or PilV, respectively. That is to say, the fragment is capable of raising an immune response with antibodies that recognizes PilE or PilV. Preferably, the fragment is capable of raising an immune response with antibodies that prevent meningococcal infection. More preferably, said fragment is capable of raising an immune response with antibodies that prevent meningococci to cross of the BBB or to spread into the meningeal space. Still more preferably, said fragment is capable of raising an immune response with antibodies that inhibit the interaction between PilE and/or PilV and β2AR.

In a particular preferred embodiment, said immunogenic fragment does not carry the hydrophobic domain of PilE or PilV. More particularly, said immunogenic fragment of PilE does not carry the hydrophobic domain of sequence FTLIELMIVIAIVGILAAVALPAYQDYTARAQVSEAILLAEGQKSAVTEYYL (SEQ ID NO: 16). More particularly, said immunogenic fragment of PilV does not carry the hydrophobic domain of sequence FTLLELMIAVAILGILTLITYPSYKTYIRRVRLSEVRTTLLHNAQTMERYYRQ (SEQ ID NO: 17).

In another particular preferred embodiment, said immunogenic fragment or PilE comprises or consists of :
a) the sequence (SEQ ID NO :18), or
b) a fragment of at least 10, 15, 20, 25, 30, 35, 40, 45, 50, 55, 60, 65, 70, 75, 80, 85, 90, 95, 100, 105, 110, 115 consecutives amino acids of the sequence SEQ ID NO :18, or
c) a sequence that is at least 80, 85, 90, 95 or 99% identical to the sequence of (a) or (b).

In another particular preferred embodiment, said immunogenic fragment or PilV comprises or consists of :
a) the sequence TMERYYRQKGTFKTYDKNKLKQNKYFNVTLSKVSPDHFTLQADPNPTTNDGETCVVTLD GGTIAASGTNQSCPGFD (SEQ ID NO :19), or
b) a fragment of at least 10, 15, 20, 25, 30, 35, 40, 45, 50, 55, 60, 65, 70, 75 consecutives amino acids of the sequence SEQ ID NO :19, or
c) a sequence that is at least 80, 85, 90, 95 or 99% identical to the sequence of (a) or (b).

In still another particular preferred embodiment, said immunogenic fragment carries the recognition domain to the β2AR responsible for meningococcal signaling.

More particularly, said immunogenic fragment of PilE comprises or consists of:
a) the sequence CGQPVTRTTATATDVAAANGKTDDKINTKHLPSTC (SEQ ID NO: 20), or
b) a fragment of at least 10, 15, 20, 25, 30 consecutives amino acids of the sequence SEQ ID NO :20, or
c) a sequence that is at least 80, 85, 90, 95 or 99% identical to the sequence of (a) or (b).

More particularly, said immunogenic fragment of PilV comprises or consists of:
a) the sequence GETCVVTLNDGGTIAASGTNQSCPGFD (SEQ ID NO:21), or
b) a fragment of at least 10, 15, 20, 25 consecutives amino acids of the sequence SEQ ID NO :21, or
c) a sequence that is at least 80, 85, 90, 95 or 99% identical to the sequence of (a) or (b).

By "meningococcal vaccine antigen" is meant a meningococcal antigen that is capable of raising an immune response against *N. meningitidis*, and that is suitable for use as a vaccine. As used herein, this term does not include PilE, PilV and immunogenic fragments thereof.

Such meningococcal vaccine antigens are well-known to the skilled in the art. Indeed, several vaccines against meningococci are commercialized and/or under development.

For example, the meningococcal vaccine antigen may correspond to one of the polysaccharide or glycoconjugate antigens comprised within the MCV-4 (commercialized under the name of Menactra® or Menveo®), MPSV-4 (Menomune®), Mencevax® or NmVac4-A/C/Y/W-135 vaccines.

The meningococcal vaccine antigen may also correspond to the capsule of group B meningococci (MenB) and/or to the outer membrane vesicle (OMV).

Preferably, the meningococcal vaccine antigen corresponds to a polypeptide. Several such polypeptidic antigens are currently investigated for their vaccinal potency. Thus the meningococcal vaccine antigen may for example be the Meningococcal Factor H binding protein (fHbp, previously referred to as GNA1870 or Lp2086), the major immunodominant surface porin (PorA), Neisserial Heparin Binding Antigen (NHBA, previously referred to as GNA2132), NadA, or immunogenic fragments thereof.

By "fHBP", it is meant a fHBP polypeptide of subfamilyB/Variant1 or of subfamilyA/Variant 2. More particularly, said fHBP polypeptide may have the sequence SEQ ID NO: 22 or SEQ ID NO: 23 or a sequence at least 80, 85, 90, 95 or 99% identical to the sequence SEQ ID NO: 22 or SEQ ID NO: 23, or an immunogenic fragment thereof.

By "PorA", it is meant a polypeptide that may have the sequence SEQ ID NO: 24 or a sequence at least 80, 85, 90, 95 or 99% identical to the sequence SEQ ID NO: 24, or an immunogenic fragment thereof.

By "NHBA", it is meant a polypeptide that may have the sequence SEQ ID NO: 25 or a sequence at least 80, 85, 90, 95 or 99% identical to the sequence SEQ ID NO: 25, or an immunogenic fragment thereof.

By "NadA", it is meant a polypeptide that may have the sequence SEQ ID NO: 26 or a sequence at least 80, 85, 90, 95 or 99% identical to the sequence SEQ ID NO: 26, or an immunogenic fragment thereof.

"Immunogenic composition" as used in the context of the present invention relates to a composition comprising at least one protein or fragment thereof (hereafter referred to as "antigen") which provokes or immunomodulates (*i.e.* immunosuppress or immunostimulate) an immune response when administered to an individual or which induces the production of antibodies when administered to an individual.

A "vaccine composition" as used herein refers to a composition, such as the immunogenic composition described herein which is administered to immunomodulate an immune response, that will protect or treat an individual from illness, in particular due to that agent. The vaccine of the present invention is intended for use both as a therapeutic (treatment) vaccine, *i.e.* for administration to the individual after development of the disease with the intention to reduce or arrest disease progression and as a preventive (prophylactic) vaccine, for administration to the individual prior to the development of the disease, with the intent to prevent initial (and/or recurrent) infection.

By "subject" or "individual" it is meant a human, more preferably a men, a woman or a child.

### Vaccines according to the invention

It has been shown that meningococcal PilE and PilV proteins are essential for *N*. *meningococcus* to cross the BBB and therefore to spread into the meningeal space. As a consequence, PilE, PilV and immunogenic fragments thereof can be used as a vaccine for preventing meningococcal infections. Indeed, administration of PilE, PilV and immunogenic fragments thereof will induce the production of antibodies inhibiting the interaction of PilE and/or PilV with β2AR, thereby preventing meningococci to cross of the BBB and to spread into the meningeal space. Therefore, an aspect of the invention provides a protein that comprises or consists of:
(a) the meningococcal PilV sequence SEQ ID NO: 2 or a sequence at least 80% identical to SEQ ID NO:2, or
(b) an immunogenic fragment of (a) that comprises or consists of SEQ ID N°21, or
(c) an immunogenic fragment of (a) that comprises or consists of a sequence that is at least 80% identical to SEQ ID NO: 21, or
(d) an immunogenic fragment of (a) that comprises or consists of SEQ ID N°19, or
(e) an immunogenic fragment of (a) that comprises or consists of a sequence that is at least 80% identical to SEQ ID N°19;
for use as a vaccine for preventing the spreading of meningococci into the meningeal space, wherein said vaccine induces the production of protective antibodies inhibiting the interaction between PilV and mammalian β2 adrenoreceptor.

In the frame of this aspect of the invention, the fragments of PilV are immunogenic fragments.

Methods for determining whether a fragment is immunogenic are well-known to the skilled in the art. For example, the capacity to raise an immune response against PilV may be tested using the assay described by Danve et al. (Vaccine. 1993;11:1214-20). Alternatively, the fragment to be tested may be used to raise specific antisera in a non-human animal such as a mouse. It may then be tested whether these antibodies are capable of inhibiting the interaction between meningococci and β2AR *in vitro*, e.g. using one of the protocols disclosed in the Examples.

As immediately apparent to the skilled in the art, in the frame of the aspect of the invention relating to vaccines, the fragments of PilV do not need to be capable of activating a mammalian β2 adrenoceptor. It is sufficient for them to be immunogenic. Preferably, the fragments of PilV need to be capable of raising an immune response with antibodies that prevent meningococcal infection, or that prevent meningococci to cross of the BBB or to spread into the meningeal space. More preferably, the fragments of PilV need to be capable of inducing the production of protective antibodies, *i.e.* antibodies inhibiting the interaction between PilV and β2AR.

The vaccine according to the invention is particularly useful for administration to individuals that are suspected of suffering from a meningococcal infection, but in whom the meningococci have not yet invaded the meningeal space. Such individuals include for example:
- individuals suspected of presenting symptoms of a meningococcal infection;
- individuals in which meningococci have been detected in the bloodstream but not in the cerebrospinal fluid;
- individuals (preferably without symptoms of a meningococcal infection) who have traveled or who reside in countries with endemic or epidemic meningococcal disease (including Europe, the USA, China and Japan); and
- individuals (preferably without symptoms of a meningococcal infection) who have been in contact with individuals with invasive meningococcal disease (e.g. relatives, schoolmates, medical staff, *etc.*).

In addition, PilV existing being present on all meningococcal groups and serotypes, the vaccine according to the invention is expected to prevent spreading of all meningococci, in particular of meningococci of group B.

PilV and immunogenic fragments thereof can be used in combination with a second meningococcal vaccine antigen.

Moreover, the invention pertains to a combination of at least two of:
- a protein that comprises or consists of :
   (i) the meningococcal PilV sequence SEQ ID NO: 2 or a sequence at least 80% identical to SEQ ID NO:2, or
   (ii) an immunogenic fragment of (i) that comprises or consists of SEQ ID N°21, or
   (iii) an immunogenic fragment of (i) that comprises or consists of a sequence that is at least 80% identical to SEQ ID NO: 21, or
   (iv) an immunogenic fragment of (i) that comprises or consists of SEQ ID N°19, or
   (v) an immunogenic fragment of (i) that comprises or consists of a sequence that is at least 80% identical to SEQ ID N°19;; and
- one or more meningococcal vaccine antigen(s);
for simultaneous or sequential use as a vaccine for preventing a meningococcal infection,wherein said vaccine induces the production of protective antibodies inhibiting the interaction between PilV and mammalian β2 adrenoreceptor, wherein said meningococcal vaccine antigen is not a PilE, PilV, or an immunogenic fragment thereof.

The combinations according to the invention may for example comprise or consist of:
- a protein that comprises or consists of :
   (i) the meningococcal PilV sequence SEQ ID NO: 2 or a sequence at least 80% identical to SEQ ID NO:2, or
   (ii) an immunogenic fragment of (i) that comprises or consists of SEQ ID N°21, or
   (iii) an immunogenic fragment of (i) that comprises or consists of a sequence that is at least 80% identical to SEQ ID NO: 21, or
   (iv) an immunogenic fragment of (i) that comprises or consists of SEQ ID N°19, or
   (v) an immunogenic fragment of (i) that comprises or consists of a sequence that is at least 80% identical to SEQ ID N°19; and
- one or more meningococcal vaccine antigen.

For example, said combination comprises or consists of the immunogenic fragment of PilV of sequence SEQ ID NO: 19 and the fHBP polypeptide of sequence SEQ ID NO: 23, or of the immunogenic fragment of PilV of sequence SEQ ID NO: 21 and the fHBP polypeptide of sequence SEQ ID NO: 23.

The said meningococcal vaccine antigens generally correspond to antigens having bactericidal activity, *i.e.* they which aim at generating an immune response with antibodies aiming at killing the meningococci. The combination according to the invention is expected to be a highly efficient vaccine since it would have two different actions: a bactericidal activity on the one hand (thanks to the meningococcal vaccine antigen), and prevention of the spreading of meningococci into the meningeal space on the other hand (thank to PilV or the immunogenic fragment thereof).

A particularly preferred aspect of the invention pertains to fusion proteins comprising at least two of:
- a protein that comprises or consists of :
   (i) the meningococcal PilV sequence SEQ ID NO: 2 or a sequence at least 80% identical to SEQ ID NO:2, or
   (ii) an immunogenic fragment of (i) that comprises or consists of SEQ ID N°21, or
   (iii) an immunogenic fragment of (i) that comprises or consists of a sequence that is at least 80% identical to SEQ ID NO: 21, or
   (iv) an immunogenic fragment of (i) that comprises or consists of SEQ ID N°19, or
   (v) an immunogenic fragment of (i) that comprises or consists of a sequence that is at least 80% identical to SEQ ID N°19; and
- one or more meningococcal vaccine antigen(s),
wherein said meningococcal vaccine antigen is not PilE, PilV, or an immunogenic fragment thereof.

Such fusion proteins are believed to be particularly suitable for use as a vaccine for preventing a meningococcal infection. Indeed, since they comprise at least two antigens that are fused together, they are highly immunogenic and thus highly efficient vaccines. In addition, they have the same advantages as the above combinations. More particularly, said fusion proteins have the ability to raise an immune response with antibodies that prevent meningococcal infection, or that prevent meningococci to cross of the BBB or to spread into the meningeal space. Still more particularly, said fusion proteins induce the production of protective antibodies inhibiting the interaction between PilV and β2AR.

Such a fusion protein according to the invention may for example comprise or consist of:
- a protein that comprises or consists of :
   (i) the meningococcal PilV sequence SEQ ID NO: 2 or a sequence at least 80% identical to SEQ ID NO:2, or
   (ii) an immunogenic fragment of (i) that comprises or consists of SEQ ID N°21, or
   (iii) an immunogenic fragment of (i) that comprises or consists of a sequence that is at least 80% identical to SEQ ID NO: 21, or
   (iv) an immunogenic fragment of (i) that comprises or consists of SEQ ID N°19, or
   (v) an immunogenic fragment of (i) that comprises or consists of a sequence that is at least 80% identical to SEQ ID N°19, fused in frame with a meningococcal vaccine antigen.

In another preferred embodiment, said combination comprises or consists of the immunogenic fragment of PilV of sequence SEQ ID NO: 19 fused with the fHBP polypeptide of sequence SEQ ID NO: 23, or of the immunogenic fragment of PilV of sequence SEQ ID NO: 21 fused with the fHBP polypeptide of sequence SEQ ID NO: 23. Consequently said fusion protein comprises or consists of the sequence SEQ ID NO: 29 or SEQ ID NO: 30.

The fusion protein according to the invention may further comprise other moieties such as e.g. peptidic linkers allowing linking the different antigens, proteins or fragments comprised within the fusion protein, a tag useful for purification, a leader sequence, a signal peptide, one or more peptidic fragments enhancing the immunogenicity, *etc.*

The invention further provides:
- the above fusion proteins for use as a vaccine for preventing a meningococcal infection, in particular for preventing the spreading of meningococci into the meningeal space;
- nucleic acids encoding the above fusion proteins;
- vaccine compositions that comprise the above fusion proteins or nucleic acids together with a pharmaceutically acceptable carrier.

The invention also relates to a vaccine composition or immunogenic composition for use for preventing the spreading of meningococci into the meningeal space by the production of protective antibodies inhibiting the interaction between PilV and mammalian β2 adrenoreceptor, said vaccine or immunogenic composition comprising or consisting of:
- a protein that comprises or consists of:
   (a) the meningococcal PilV sequence SEQ ID NO: 2 or a sequence at least 80% identical to SEQ ID NO:2, or
   (b) an immunogenic fragment of (a) that comprises or consists of SEQ ID N°21, or
   (c) an immunogenic fragment of (a) that comprises or consists of a sequence that is at least 80% identical to SEQ ID NO: 21, or
   (d) an immunogenic fragment of (a) that comprises or consists of SEQ ID N°19, or
   (e) an immunogenic fragment of (a) that comprises or consists of a sequence that is at least 80% identical to SEQ ID N°19;
   according to the invention,
- a combination of at least two of : a protein that comprises or consists of :
   (i) the meningococcal PilV sequence SEQ ID NO: 2 or a sequence at least 80% identical to SEQ ID NO:2, or
   (ii) an immunogenic fragment of (i) that comprises or consists of SEQ ID N°21, or
   (iii) an immunogenic fragment of (i) that comprises or consists of a sequence that is at least 80% identical to SEQ ID NO: 21, or
   (iv) an immunogenic fragment of (i) that comprises or consists of SEQ ID N°19, or
   (v) an immunogenic fragment of (i) that comprises or consists of a sequence that is at least 80% identical to SEQ ID N°19;
      and a meningococcal vaccine antigen, according to the invention, or
- a fusion protein according to the invention.

More particularly, said vaccine composition or immunogenic composition comprises or consists of a combination comprising or consisting of at least two of:
- a protein that comprises or consists of :
   (i) the meningococcal PilV sequence SEQ ID NO: 2 or a sequence at least 80% identical to SEQ ID NO:2, or
   (ii) an immunogenic fragment of (i) that comprises or consists of SEQ ID N°21, or
   (iii) an immunogenic fragment of (i) that comprises or consists of a sequence that is at least 80% identical to SEQ ID NO: 21, or
   (iv) an immunogenic fragment of (i) that comprises or consists of SEQ ID N°19, or
   (v) an immunogenic fragment of (i) that comprises or consists of a sequence that is at least 80% identical to SEQ ID N°19; and
- a meningococcal vaccine antigen,
wherein said meningococcal vaccine antigen is not PilE or PilV or an immunogenic fragment thereof.

For example, said vaccine composition or immunogenic composition comprises or consists of a combination comprising or consisting of the immunogenic fragment of PilV of sequence SEQ ID NO: 19 and the fHBP polypeptide of sequence SEQ ID NO: 23, or of the immunogenic fragment of PilV of sequence SEQ ID NO: 21 and the fHBP polypeptide of sequence SEQ ID NO: 23.

Still more particularly, said vaccine composition or immunogenic composition comprises or consists of a fusion protein comprising or consisting of at least two of:
- a protein that comprises or consists of :
   (i) the meningococcal PilV sequence SEQ ID NO: 2 or a sequence at least 80% identical to SEQ ID NO:2, or
   (ii) an immunogenic fragment of (i) that comprises or consists of SEQ ID N°21, or
   (iii) an immunogenic fragment of (i) that comprises or consists of a sequence that is at least 80% identical to SEQ ID NO: 21, or
   (iv) an immunogenic fragment of (i) that comprises or consists of SEQ ID N°19, or
   (v) an immunogenic fragment of (i) that comprises or consists of a sequence that is at least 80% identical to SEQ ID N°19 and
- one or more meningococcal vaccine antigen(s),
wherein said one or more meningococcal vaccine antigen(s) is not PilE or PilV or an immunogenic fragment thereof.

For example, said vaccine composition or immunogenic composition comprises or consists of a fusion protein comprising or consisting of the immunogenic fragment of PilV of sequence SEQ ID NO: 19 fused with the fHBP polypeptide of sequence SEQ ID NO: 23, or of the immunogenic fragment of PilV of sequence SEQ ID NO: 21 fused with the fHBP polypeptide of sequence SEQ ID NO: 23. Accordingly, said vaccine composition or immunogenic composition comprises or consists of a fusion protein comprising or consisting of the sequence SEQ ID NO: 29 or SEQ ID NO: 30.

Methods of obtaining a vaccine composition or immunogenic composition are well known in the art. Generally such methods involve extracting proteins from bacterial preparations using techniques such as sonication, proteolytic digestion, heat treatment, freeze-thaw treatment, osmotic shock treatment etc.... Examples of artificial bacterial preparations include protein preparations either in part or entirely obtained by synthetic or recombinant methods. Oligonucleotides can probes can be devised based on the sequences of the bacterial genome and can be used to probe genomic or cDNA libraries for genes encoding antigens useful in the context of the invention. Genes can be isolated using standard techniques.

The vaccine composition or immunogenic composition can comprise a suitable adjuvant. The type of adjuvant will vary, depending on the type of antigen preparation and route of administration used. Any adjuvant known in the art may be used in the immunogenic composition, including oil-based adjuvants such as Freund's Complete Adjuvant and Freund's Incomplete Adjuvant, mycolate-based adjuvants (e.g., trehalose dimycolate), bacterial lipopolysaccharides (LPS), peptidoglycans (*i.e.,* mureins, mucopeptides, or glycoproteins such as N-Opaca, muramyl dipeptide [MDP], or MDP analogs), proteoglycans (e.g., extracted from Klebsiella pneumoniae), streptococcal preparations (e.g., OK432), Biostim.TM. (e.g., 01 K2), the "Iscoms" of EP 109 942, EP 180 564 and EP 231 039, aluminum hydroxide, saponin, DEAE-dextran, neutral oils (such as miglyol), vegetable oils (such as arachis oil), liposomes, Pluronic.RTM. polyols. Adjuvants include, but are not limited to, the RIBI adjuvant system (Ribi Inc.), alum, aluminum hydroxide gel, cholesterol, oil-in water emulsions, water-in-oil emulsions such as, e.g., Freund's complete and incomplete adjuvants, Block co-polymer (CytRx, Atlanta Ga.), SAF-M (Chiron, Emeryville Calif.), AMPHIGEN.RTM. adjuvant, saponin, Quil A, QS-21 (Cambridge Biotech Inc., Cambridge Mass.), GPI-0100 (Galenica Pharmaceuticals, Inc., Birmingham, Ala.) or other saponin fractions, monophosphoryl lipid A, Avridine lipid-amine adjuvant, heat-labile enterotoxin from E. coli (recombinant or otherwise), cholera toxin, or muramyl dipeptide, among many others.

Suitable pharmaceutically acceptable carrier is for example an adjuvant. Adjuvants include, but are not limited to oil-based adjuvants such as Freund's Complete Adjuvant and Freund's Incomplete Adjuvant, mycolate-based adjuvants (*e.g*., trehalose dimycolate), bacterial lipopolysaccharides (LPS), peptidoglycans (*i.e.,* mureins, mucopeptides, or glycoproteins such as N-Opaca, muramyl dipeptide [MDP], or MDP analogs), proteoglycans (*e.g.,* extracted from Klebsiella pneumoniae), streptococcal preparations (e.g., OK432), Biostim.TM. (*e.g*., 01 K2), the "Iscoms" of EP 109 942, EP 180 564 and EP 231 039, aluminum hydroxide, saponin, DEAE-dextran, neutral oils (such as miglyol), vegetable oils (such as arachis oil), liposomes, Pluronic.RTM. polyols, the RIBI adjuvant system (Ribi Inc.), alum, aluminum hydroxide gel, cholesterol, oil-in water emulsions, water-in-oil emulsions such as, e.g., Freund's complete and incomplete adjuvants, Block co-polymer (CytRx, Atlanta Ga.), SAF-M (Chiron, Emeryville Calif.), AMPHIGEN.RTM. adjuvant, saponin, Quil A, QS-21 (Cambridge Biotech Inc., Cambridge Mass.), GPI-0100 (Galenica Pharmaceuticals, Inc., Birmingham, Ala.) or other saponin fractions, monophosphoryl lipid A, Avridine lipid-amine adjuvant, heat-labile enterotoxin from E. coli (recombinant or otherwise), cholera toxin, or muramyl dipeptide, among many others.

The immunogenic composition, the vaccines and the fusion proteins according to the invention can be administered via any suitable route, such as by mucosal (intranasal), parenteral, or intramuscular administration, oral, intradermal, intraperitoneal, intravenous, or subcutaneous administration. Suitable vaccination routes also comprise combination administrations (*e.g.* oral/intramuscular administration).

The immunogenic composition, the vaccines and the fusion proteins according to the invention can be administered alone or with suitable pharmaceutical carriers, and can be in solid or liquid form such as, tablets, capsules, powders, solutions, suspensions, or emulsions.

The solid unit dosage forms can be of the conventional type. The solid form can be a capsule, such as an ordinary gelatin type containing the proteins or peptides of the present invention or the antibodies or binding portions thereof of the present invention and a carrier, for example, lubricants and inert fillers such as, lactose, sucrose, or cornstarch. In another embodiment, these compounds are tableted with conventional tablet bases such as lactose, sucrose, or corn starch in combination with binders like acacia, corn starch, or gelatin, disintegrating agents such as, corn starch, potato starch, or alginic acid, and a lubricant like stearic acid or magnesium stearate.

The immunogenic composition, the vaccines and the fusion proteins according to the invention may also be administered in injectable dosages by solution or suspension of these materials in a physiologically acceptable diluent with a pharmaceutical carrier. Such carriers include sterile liquids such as water and oils, with or without the addition of a surfactant and other pharmaceutically acceptable adjuvants. Illustrative oils are those of petroleum, animal, vegetable, or synthetic origin, for example, peanut oil, soybean oil, or mineral oil. In general, water, saline, aqueous dextrose and related sugar solution, and glycols such as, propylene glycol or polyethylene glycol, are preferred liquid carriers, particularly for injectable solutions.

For use as aerosols, the immunogenic composition, the vaccines and the fusion proteins according to the invention of the present invention in solution or suspension may be packaged in a pressurized aerosol container together with suitable propellants, for example, hydrocarbon propellants like propane, butane, or isobutane with conventional adjuvants. The materials of the present invention also may be administered in a non-pressurized form such as in a nebulizer or atomizer.

Although having distinct meanings, the terms "comprising", "having", "containing' and "consisting of" have been used interchangeably throughout this specification and may be replaced with one another.

In the frame of the present application, all agents, molecules, compounds, proteins, polypeptides, peptides, nucleic acids, vectors and host cells may be isolated and/or purified.

The invention will be further evaluated in view of the following examples and figures.

### BRIEF DESCRIPTION OF THE FIGURES

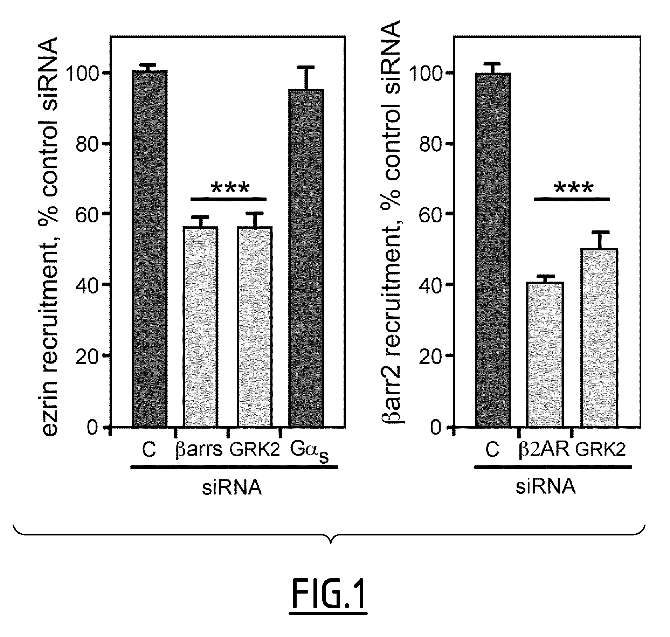

**Figure 1****. Requirement of GRK2 and βarrs for *Nm*-induced ezrin honeycombs.**
   Left panel: Effect of siRNA-dependent inhibition of βarrs, GRK2, and Gas expression on ezrin recruitment under *Nm* colonies; the recruitment assay is described in Experimental Procedures. Data are the average values (±SEM) of ezrin recruitment compared to the control (scramble siRNA-treated cells) from 3 independent experiments in triplicate. ***(p<0.001). Right panel: Effect of siRNA-dependent inhibition of β2AR and GRK2 expression on βarr2 recruitment under *Nm* colonies; the recruitment assay is as in (**c**) except that the readout is based on βarr2-GFP accumulation.

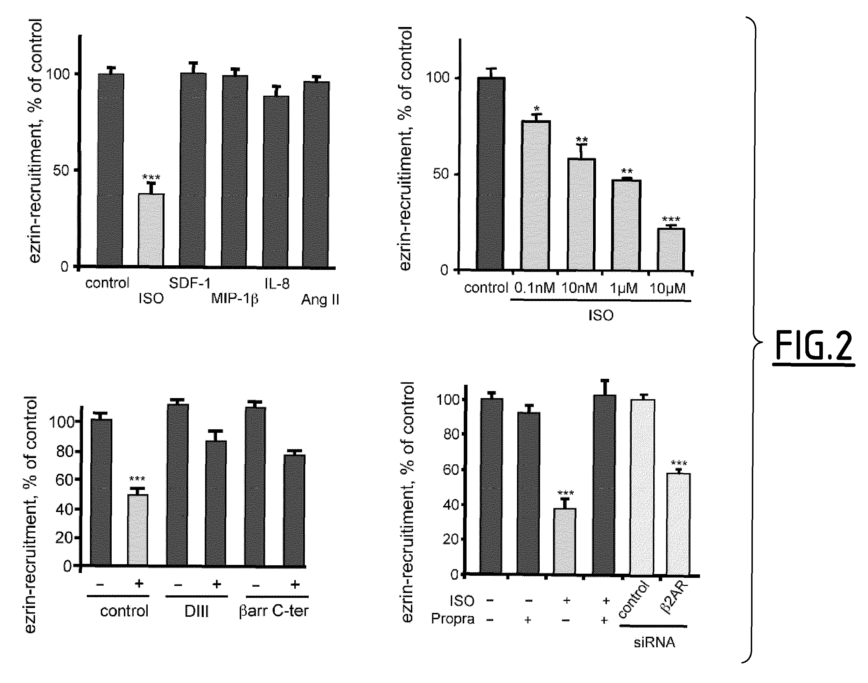

**Figure 2****. β2AR involvement in ezrin recruitment to *Nm* colonies.**
   Top left panel: Effect of pre-treatment with saturating concentrations of specific GPCR agonists on *Nm*-induced ezrin recruitment in hCMEC/D3 cells. ISO: β2AR agonist isoproterenol, 5µM; SDF-1: CXCR4 agonist, 100nM; MIP-1β: CCR5 agonist, 100nM; IL-8: CXCR1-2 agonist, 100nM; Ang II: angiotensin II, ATIR agonist, 10nM. ***(p<0.001). Top right panel: Dose-dependent effect of isoproterenol on *Nm*-promoted ezrin recruitment. Bottom left panel: Effect of endocytosis inhibitors on isoproterenol modulation of *Nm-*promoted ezrin recruitment. hCMEC/D3 cells were transfected with plasmids for either DIII (dominant negative mutant of Eps15 fused to GFP) or βarrC-ter (carboxyterminal tail of βarr fused to GFP), 24h before isoproterenol pre-treatment (+) and subsequent ezrin recruitment assay; (-) control cells pre-treated with medium alone; ***(p<0.001). Bottom right panel: Reversal of the effect of isoproterenol pre-treatment on *Nm*-promoted ezrin recruitment by propranolol and inhibition of ezrin honeycombs by pre-treating hCMEC/D3 cells with β2AR specific siRNA. ISO: 10µM isoproterenol, Propra: 100nM propranolol; ***(p<0.001)

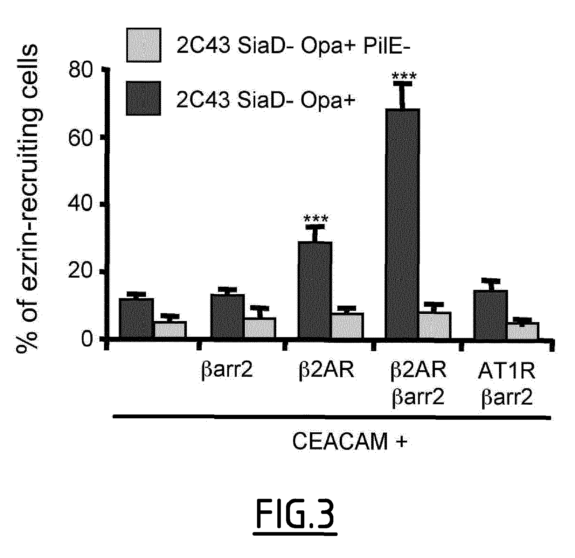

**Figure 3****. Reconstitution of *Nm*-promoted signaling in HEK-293 cells**
   Ezrin recruitment assay in HEK-293 cells transfected with the indicated plasmids and infected with the indicated *Nm* strains. AT1 R: control Type 1 Ang II receptor. Bars indicate SEM from 3 experiments in duplicate. ***p<0.001. Other control receptors were tested (CCR5, CXCR4), which all failed to reconstitute ezrin recruitment above the values observed for AT1 R (not shown).

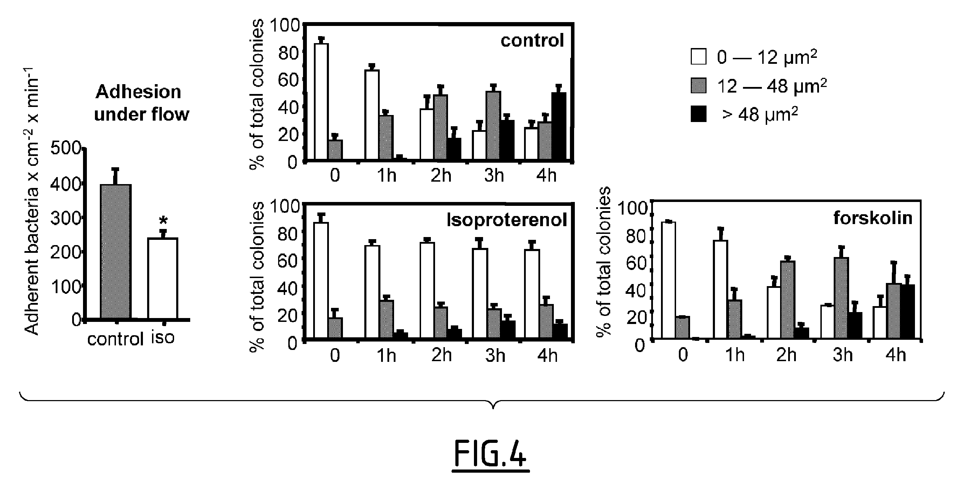

**Figure 4****. Effect of isoproterenol on *Nm* adhesion and growth under flow**
   Left panel: "Under flow" adhesion of bacteria on hCMEC/D3 cells pre-treated with 10µM isoproterenol or left untreated. Adhesion under shear stress was determined by infecting cells grown in IBIDI™ flow chamber under a shear stress of 0.04 dyn/cm² as described in Experimental Procedures. No significant difference was observed for static adhesion in the absence or presence of isoproterenol (p=0,73, n=6), whereas after pre-treatment with the β2AR agonist, "under flow" studies showed a 40% reduction of bacterial adhesion (*p=0.018, n=6). Right panel: Analysis of the size of *Nm* colonies growing on hCMEC/D3 cells under flow in control conditions or after cell pretreatment with 10µM isoproterenol or forskolin for 1 h before injection of bacteria. Colonies were classified in 3 groups according to their size for each indicated time (see Experimental Procedures for details).

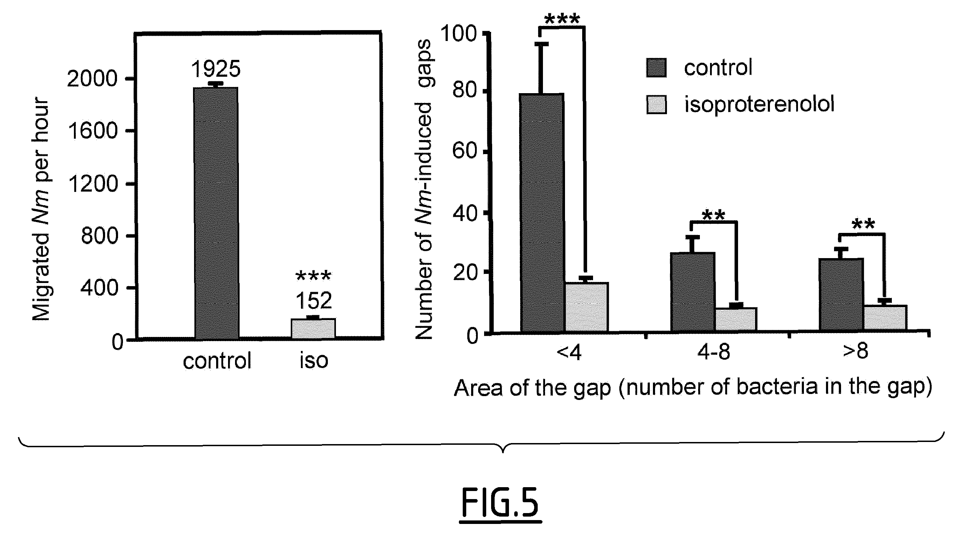

**Figure 5****. *Nm* crossing of hCMEC/D3 cell monolayers.**
   Left panel: Transmigration assays of *Nm* on hCMEC/D3 cells grown on 3µm pore size MilliCell® inserts. Transmigration in the presence or absence of isoproterenol pretreatment (1 h, 10µM) was expressed as the number of migrating bacteria through the cell monolayer per hour. Data correspond to 5 independent experiments in duplicate; ***p<0.001. Right panel: Surface and number of cell-cell junction gaps induced by *Nm,* with or without isoproterenol pretreatment (10µM, 1 h), visualized by confocal fluorescence microscopy on fixed MilliCell® inserts immunostained for VE-cadherin. The areas of gaps (see experimental procedures) were classified in 3 groups according to the number of bacteria that can be accommodated in the gap: 4 diplococci or less, 4 to 8 diplococci, more than 8 diplococci ***p<0.001, **p<0.01. The total surface occupied by bacteria on the apical surface of the monolayer, determined by anti-pili staining, was similar with or without isoproterenol (not shown).

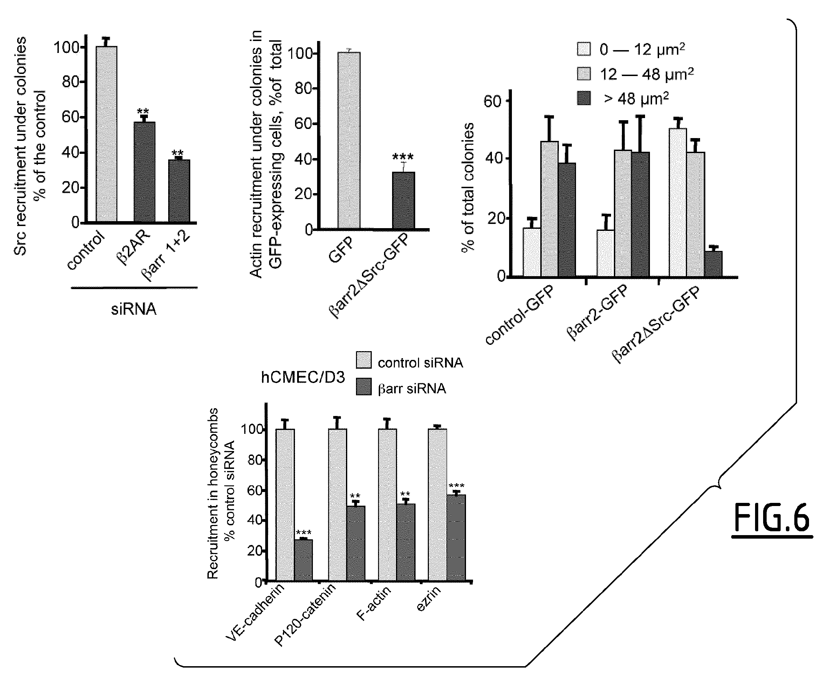

**Figure 6****. Role of βarrs in *Nm*-promoted signaling.**
   Top panels: Docking and activation of Src. Top left panel: Reduction of Src recruitment under *Nm* colonies in hCMEC/D3 cells treated with β2AR or βarr(1+2) siRNAs; n=6, **p<0.01 Top middle panel: Dominant negative effect of βarr2-□Src-GFP on actin recruitment in the same cells. Details on βarr2-ΔSrc construct are in Supplemental Methods. n=6, ***p<0.001 Top right panel: Size of *Nm* colonies growing on hCMEC/D3 cell monolayers under laminar flow in control GFP-transfected cells, and in cells expressing either barr2-GFP or dominant negative βarr2-ΔSrc-GFP. Bottom panel: βarr-dependent recruitment of junctional proteins into cortical plaques. Effect of the siRNA-mediated inhibition of βarr1 and βarr2 gene expression on junctional and cytoskeletal protein recruitment into cortical plaques in *Nm*-infected hCMEC/D3 cells. Data correspond to average values (±SEM) of 3 independent experiments in duplicate. ***p<0.001, **p<0.01 relative to control.

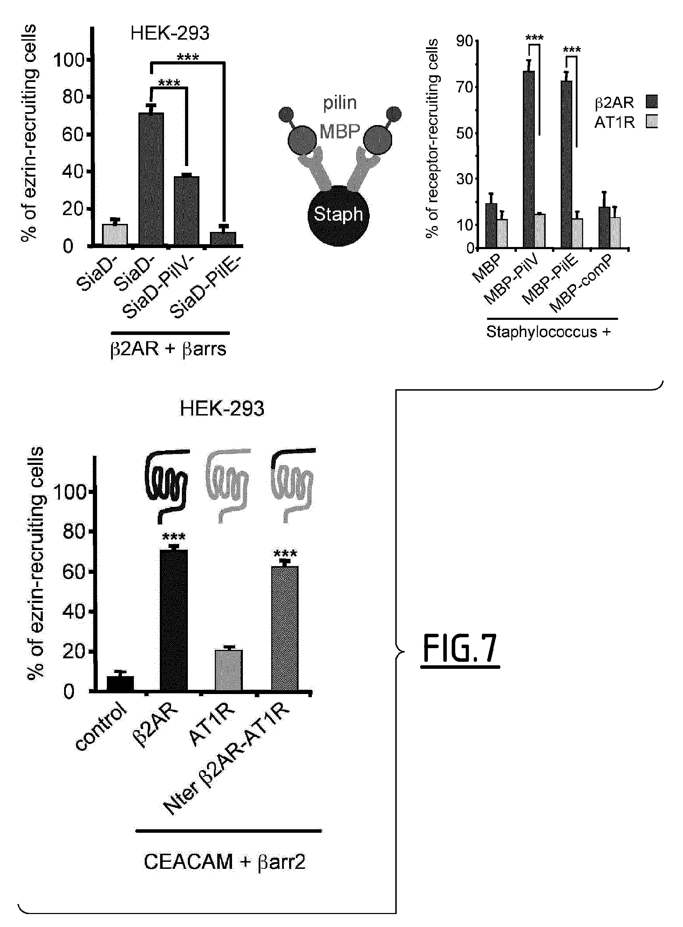

**Figure 7****. Molecular determinants of *Nm*-β2AR interaction**
   Top left panel: Ezrin recruitment assay in wt or β2AR- and βarr-reconstituted Hek-293 cells (as in Figure 3) infected with a piliated SiaD- Opa+ *Nm* strain displaying normal or PilV- pili or with a non-piliated SiaD- Opa+ strain (PilE-); ***p<0.001. Top right panel: The upper left cartoon depicts the approach to examine the respective role of pilins. Receptor recruitment was counted in all cells in contact with staphylococci aggregates; data correspond to average values (±SEM) of 3 independent experiments in duplicate. ***p<0.001, **p<0.01. Bottom panel: Ezrin recruitment experiment comparing the β2AR the AT1R and the chimeric Nterβ2AR-AT1R; ***p<0.001 compared to control and AT1R.

### BRIEF DESCRIPTION OF THE SEQUENCE LISTING

SEQ ID NO: 1 shows the amino acid sequence of PilE.
SEQ ID NO: 2 shows the amino acid sequence of PilV.
SEQ ID NO: 3 shows the amino acid sequence of β2AR. The position of the N-terminus present in the Nterβ2AR-AT1R construct is indicated.
SEQ ID Nos. 4 to 7 show the sequences of siRNAs used in Example 1.9.
SEQ ID Nos. 8 to 11 show the sequences of primers used in Example 1.16.
SEQ ID Nos. 12 to 15 show the sequences of primers used in Example 1.18.
SEQ ID NO: 16 shows the sequence of the hydrophobic domain of PilE.
SEQ ID NO: 17 shows the sequence of the hydrophobic domain of PilV.
SEQ ID NOs: 18 and 20 show the sequences of immunogenic fragments of PilE.
SEQ ID NOs: 19 and 21 show the sequences of immunogenic fragments of PilV.
SEQ ID NOs: 22 and 23 show the sequences of the fHBP polypeptides.
SEQ ID NO: 24 shows the sequence of the PorA polypeptide.
SEQ ID NO: 25 shows the sequence of the NHBA polypeptide.
SEQ ID NO: 26 shows the sequence of the NadA polypeptide.
SEQ ID NOs: 27 and 28 show the sequences of the fusion proteins between PilE and fHBP polypeptide (is not part of the invention).
SEQ ID NOs: 29 and 30 shows the sequences of the fusion proteins between PilV and fHBP polypeptide.

### EXAMPLES

### EXAMPLE 1: Protocols and experimental procedures

### 1.1. Cell line and Bacterial Strains

The hCMEC/D3 cell line, retaining the main characteristics of primary brain endothelial cells, was described previously (Weksler et al., 2005, FASEB J 19, 1872-1874). A piliated capsulated Opa⁻ variant of serogroup-C meningococcal strain 8013, designated 2C43 (Nassif *et al*., 1993, Mol Microbiol 8, 719-725), was used throughout the study unless otherwise specified. Non-capsulated isogenic derivatives were engineered by introducing a *siaD* mutation. Isogenic non-piliated, piliated or PilV- variants of an Opa⁺ non-capsulated 2C43 derivative were designated SiaD- Opa⁺ PilE⁻, SiaD- Opa⁺ and SiaD-Opa⁺ PilV⁻, respectively. *Nm* expressing the green fluorescent protein (GFP) were obtained by introducing in bacteria the pAM239 plasmid by conjugation (Mairey et al., 2006, J Exp Med 203, 1939-1950).

### 1.2. Quantitative analysis of protein recruitment under bacterial colonies

The recruitment efficiency was estimated by determining the proportion of colonies positive for the indicated protein of interest. At least 50 colonies were observed per coverslip. Each experiment was repeated several times in duplicate or triplicate. Data was examined for significance using Student's *t*-test.

### 1.3. Transfections

hCMEC/D3 cells were transfected using the Amaxa Nucleofactor Kit for Huvec (Amaxa Biosystem) or jetPEI transfection reagent (Polyplus Transfection) according to the manufacturer's instructions. HEK-293 cells were transfected using Lipofectamine 2000 (Invitrogen).

### 1.4. Nm adhesion under shear stress

hCMEC/D3 cells were grown on disposable flow chambers (15µ-Slide VI, IBIDI) coated with 5µg/cm² of rat-tail collagen type I. For experiments investigating *Nm* colony growth under shear stress, 10⁷ bacteria were introduced into the flow chamber and allowed to adhere for 10min without flow. Subsequently, a flow corresponding to a shear stress of 0.2dyn/cm² was applied using a syringe pump (Harvard Apparatus). For adhesion assays under shear stress, 10⁷ bacteria were allowed to adhere for 20 min under a shear stress of 0.04dyn/cm², then the chambers were washed 1 min under a shear stress of 1dyn/cm² (Mairey et al., 2006, J Exp Med 203, 1939-1950).

### 1.5. Nm transmigration assay

hCMEC/D3 cells were grown on 3µm pore size MilliCell® inserts and infected at a MOI of 10, corresponding to 10⁶ bacteria per insert. Four hours after infection, inserts were placed in a well with fresh medium. After additional 30min, the number of bacteria, which had crossed the cell monolayer was determined by plating the content of the well and counting the number of colony forming units. To estimate the surface and number of macroscopic cell-cell junction openings, MilliCell® inserts were fixed in 4% PFA and immunostained for VE-cadherin and actin. Several baso-lateral cross-sections of each insert were acquired using a Leica SP5 confocal microscope. Surface and number of gaps within the hCMEC/D3 cell monolayer was determined using Leica Application Suite-AF (LAS-AF) software. Data were expressed as the number of gaps per 4x10⁵ µm² of hCMEC/D3 cell monolayer surface. The size of gaps was determined using the apparent size of a *Nm* diplococcus as surface unit (approximately 1.7 µm²).

### 1.6. Cell culture

The immortalized human brain micro-vascular endothelial cell line hCMEC/D3, which retains the main characteristics of primary brain endothelial cells, was described previously. hCM EC/D3 cells were grown at a density of 25x1 03 cells per cm² in flasks coated with 5µg/cm 2 rat tail collagen type I (BD Bioscience), in EBM-2 medium (Lonza) supplemented with 5% of Fetal Calf Serum, 1% of Penicillin-Streptomycin, 1.4µM hydrocortisone, 5µg/ml ascorbic acid, 10mM HEPES and 1ng/ml b-FGF, at 37°C in a humidified incubator in 5% CO2. Human Embryonic Kidney 293 (HEK-293) cells were grown in flasks coated with 0.01% poly-L-lysine (Sigma), containing DMEM medium supplemented with 10% Fetal Calf Serum and 1% Penicillin-Streptomycin, in 5% CO2-enriched humidified atmosphere. For immunofluorescence assays, cells were grown on 12- mm diameter glass coverslips with appropriate coating and media. The ability of bacteria to transmigrate through hCMEC/D3 cells monolayers was assayed with Millicell® inserts of indicated pore size. Cells were seeded at a density of 5x1 04 cells per cm2 on inserts pre-coated with rat tail collagen type I (thick coating, modified from Bornstein and Murray) for 12-15 days. The medium was replaced every 3-4 days and 24h before the experiment.

### 1.7. Bacterial strains, growth and infection conditions

A piliated capsulated Opa- variant of serogroup-C meningococcal strain 8013, designated 2C43, was used throughout the study (Nassif et al., 1993, Mol Microbiol 8, 719-725). A non-capsulated derivative of this strain was engineered by introduction of a siaD mutation. Isogenic non-piliated and piliated Opa⁺ strains of this non-encapsulated 2C43 derivative were designated SiaD- Opa⁺ PilE- and SiaD- Opa⁺, respectively (Coureuil et al., 2009, Science 325, 83-87). Isogenic piliated and non-capsulated Opa+ mutant for the pilV gene was designated SiaD- Opa+ PilV- (Mikaty *et al*., 2009, PLoS Pathog 5, e1000314). N. meningitidis expressing the green fluorescent protein (GFP) were obtained by introducing in bacteria the pAM239 plasmid by conjugation (Mairey *et al*., 2006, J Exp Med 203, 1939-1950). The day before the infection, hCMEC/D3 cells were serum-starved in EBM-2 medium containing 0.1% Bovine Serum albumin (BSA). The day of infection, bacteria from an overnight culture were adjusted to OD600=0.05 and incubated for 2h at 37'C in pre-warmed starvation medium. Bacteria were added to cells at a multiplicity of infection (MOI) of 100 and allowed to adhere for 30 minutes. Unbound bacteria were washed with fresh medium and infection was al lowed to proceed for 2h. When needed, drugs were added to cell culture 1 h before infection. Bacteria were tested for growth and aggregation. Bacterial colonies harvested from GCB agar plates were grown to OD600=0.05 in EBM2, 0.1 % BSA in the presence or Absence of 100µM isoproterenol and incubated at 37'C in a 5% CO2 atmosphere under constant shaking. The OD600 was then measured after 1, 2, 3, 4 and 8h. Aggregation was determined after 2h of incubation in EBM2, 0.1% BSA in the presence or absence of 100µM isoproterenol at 37°C in a 5% CO2 atmosphere under constant shaking. A cone and plate assay was then performed as described previously (Mikaty *et al*., 2009) using a cone and plate rheometer (Brookfield Engineering Laboratories Inc., Middleboro, MA, U SA).

### 1.8. Plasmids and transfections

Plasmids coding for βarr1-GFP, βarr2-GFP, myc-βarr1 and myc- βarr2, the carboxyterminal tail of βarr2-fused to GFP, the human β2AR-YFP (β2AR-YFP) the AT1R, CCR5 and CXCR4 were described previously, as were plasmids coding for dominant negative Eps15 (Eps15-DIII-GFP) and control Eps15-DIIIΔ2-GFP, and the plasmid encoding CEACAM-1. The 4xCRE-Luciferase reporter plasmid was purchased from Stratagene. βarr2-ΔSrc (βarr2 P91G P93D, P94R, P97L) was prepared by site directed mutagenesis using βarr2-GFP or myc-βarr2 as templates and the QuikChange (Stratagene) protocol.

The chimeric Nterβ2AR-AT1R receptor was constructed using a 2-step procedure. First a unique Kpn1 site was introduced by site-directed mutagenesis in the human AT1 R immediately before the beginning of the first transmembrane domain (position F28). The N-terminus of the human β2AR (amino acids 1 to 28 of SEQ ID NO: 3), amplified by PCR, was then subcloned in phase upstream of the codon for F28 using the Kpn1 site. The constructs for β2AR-ΔSrc and Nterβ2AR-AT1R were sequenced to verify the phases and the absence of undesired mutations (DNA sequence facility, Institut Cochin). hCMEC/D3 cells were transfected using the Amaxa Nucleofactor Kit for Huvec (Amaxa Biosystem) or jetPEI transfection reagent (Polyplus Transfection) according to manufacturer instructions. HEK-293 cells were transfected using Lipofectamine 2000 (Invitrogen) following manufacturer instructions.

### 1.9. Inhibition of protein expression by RNA interference

Small interfering RNAs targeting human βarrestin-1 (5'-AAAGCCUUCUGCGCGGAGAAU-3', SEQ ID NO: 4), βarrestin-2 (5'-GGCUUGUCCUUCCGCAAAGAC-3', SEQ ID NO: 5), and GRK2 (5'-AAGAAGUACGAGAAGCUGGAGUU-3', SEQ ID NO: 6) were synthesized by Eurogentech. To silence the expression of Gas and of the β2-adrenoceptor, a pool of four siRNA duplexes (ON-Target plus SMARTpool, NM_080426 and NM_000024 from Dharmacon, respectively) were used. The efficacy of knockdown was assessed three days after transfection by immunoblotting or by RT-PCR assays using whole cell lysates. An offtarget siRNA (5'-GATAAGGTGCTGCGTGGACCC-3', SEQ ID NO: 7) and the siCONTROL siRNA (Dharmacon) were used as controls. Si RNAs were transfected using Amaxa Nucleofactor Kit for Huvec according to manufacturer's instructions. The infections with *Nm* were performed three days after transfection.

### 1.10. Antibodies, drugs and receptor ligands

The following monoclonal antibodies (mAb) or polyclonal antibodies were used for immunofluorescence labeling or immunoblotting: anti-ezrin rabbit antibody, anti-βarr rabbit A1CT antibody, rabbit monoclonal anti-βarr2 antibody (C16D9, Cell Signaling), anti-Gas rabbit antibody, anti-GRK2 mouse mAb (clone C5/1.1, Upstate), anti-src rabbit polyclonal antibody (SC-18, Santa Cruz), anti-phosphorylated Src (PY418) rabbit monoclonal antibody (Bioscience, #44-660G), anti-actin rabbit polyclonal antibody (Sigma-Aldrich), anti-VE-cadherin rabbit polyclonal antibody (Bender Medsystems), anti-p120-catenin mouse mAb (BD Transduction Laboratories), anti-myc mouse mAb or rabbit polyclonal anti body (Cell Signaling), anti -type IV pili mouse mAb 20D9 and anti-GFP mouse mAb 3E6 (Invitrogen). Following Goat secondary antibodies were used for Immunofluorescence labelling or immunoblotting: anti-mouse IgG (H+L) and anti-rabbit IgG (H+L) coupled to Horse Radish Peroxidase (Jackson ImmunoResearch Laboratories) and anti-mouse IgG (H+L) and anti-rabbit IgG (H+L) coupled to Alexa-488 or Alexa-546 or Alexa-633 (Invitrogen). Isoproterenol, propranolol, angiotensin II (Ang II) forskolin and PP2 Src inhibitor were purchased from Sigma-Aldrich. SDF-1 (agonist of CXCR4 receptor), MIP-1β (agonist of CCR5 receptor), IL8 were al I from Preprotech.

### 1.11. Immunofluorescence experiments

Cells were fixed for 20min in PBS 4% PFA and permeabilized for 5min in PBS containing 0.1% Triton X-100. Cells were incubated with primary antibodies at the recommended concentration in PBS containing 0.3% BSA for 1 h. After 3 washings in the same buffer, Alexa-conjugated phalloidin and DAPI (0.5µg/ml) were added to Alexa-conjugated secondary antibodies for 1h. After additional washings, coverslips were mounted in mowiol. Image acquisition was performed on a laser-scanning confocal microscope (Zeiss LSM 510). Images were collected and processed using the Leica Application Suite AF lite (Leica) software. 3D reconstruction was performed using the IMARIS software.

### 1.12. Quantitative analysis of ezrin (and other proteins) recruitment under bacterial colonies

After infection, cells were treated for immunofluorescence analysis with appropriate antibodies and observed under a light fluorescent microscope. For each *Nm* strain, the recruitment efficiency was estimated by determining the proportion of colonies that efficiently recruited the protein of interest in a "honeycomb shape" just under the colonies. Importantly, in uninfected cells, no ezrin, actin or Src were found in such honeycomb-shaped clusters at the plasma membrane. At least 50 colonies were scrutinized per coverslip. Each experiment was repeated several times (at least 3). Data was examined for significance using Student's t-test.

### 1.13. CRE-Luciferase assay

Luciferase activity in cell lysates was measured using the Luciferase Reporter Assay System (Promega) according to manufacturer instructions. Briefly, hCMEC/D3 cells were transfected with the 4xCRE-Luciferase plasmid (Stratagene) the day before the experiment. Cell infection was performed as described above at a MOI of 100. After 2h of infection or incubation with drugs or ligands, cells were washed twice with ice-cold PBS, and lysed using the Luciferase Cell Culture Lysis Reagent. Lysates were centrifuged at 12,000xg for 2mi n at 4°C. Supernatants were tested for luciferase expression using a V ICTOR 3 multilabel counter (Perkin-Elmer).

### 1.14. Nm adhesion under shear stress

Shear stress assays were performed as described previously (Mairey et al., 2006, J Exp Med 203, 1939-1950). Briefly, cells were grown on disposable flow chambers (15µ-Slide VI, IBIDI) coated with 5µg/cm2 rat tail collagen type I. Bacteria grown to OD600=0.05 in EBM2, 0.1 % BSA medium were incubated for 2h at 37°C under 5% CO2 atmosphere. GFP expression in bacteria was induced by adding 1 mM IPTG in EBM2, 0.1% BSA. For experiments aimed at determining the growth of colonies under shear stress, 107 bacteria were then introduced into the flow chamber and al lowed to adhere for 20 min without flow. Subsequently, a flow corresponding to a shear stress of 0.2 dyn/cm2 was applied using a syringe pump (Harvard Apparatus). For adhesion assays under shear stress, 10 bacteria were introduced into the flow chamber and al lowed to adhere for 20 min under a shear stress of 0. 04 dyn/cm2, subsequently the chamber was washed 1 min under a shear stress of 1 dyn/cm2. Fluorescent bacteria in the chambers were observed using an Olympus CKX41 inverted microscope with a 20x objective, equipped with a shutter for the fluorescence I amp and a Hamamatsu ORCA285 CCD camera. Images were collected and processed using the Openlab software (Improvision). The field under observation corresponded to a surface of 425 µm by 320 µm with a resolution of 0.63 µm per pixel. Images were analyzed using the ImageJ software.

### 1.15. Nm transmigration assay

Cell permeability to *Nm* was determined as described previously (Coureuil et al., 2009, Science 325, 83-87). Briefly, hCMEC/D3 cells were grown on 3µm pore size MilliCell® inserts and infected at a MOI of 10 (10⁶ bacteria per insert). Four hours after infection, inserts were placed in a well with fresh medium. After 30min, the number of bacteria, which had crossed the monolayer of endothelial cells, was then measured by plating the content of dilutions of the lower compartment on agar plates and counting the number of colony forming units (CFU). The effect of isoproterenol on *Nm* transmigration was determined by comparing the number of migrating bacteria through the monolayer of endothelial cells per hour in the presence and absence of the agonist. To estimate the surface and number of macroscopic cell-cell junction openings, MilliCell® inserts were fixed at the end of the experiments in 4% PFA and immunostained for V E-cadherin and actin. Several baso-lateral cross-sections of each insert were acquired using a Leica SP5 confocal microscope. The surface and number of gaps within the hCMEC/D3 cell monolayer was determined using the Leica Application Suite-AF (LAS-AF) software. Data were expressed as the number of gaps per 4.10⁵ µm² of hCMEC/D3 cell monolayer surface. The size of gaps was determined using the size of a *Nm* diplococcus as surface unit (approximately 1.7 µm²). The total surface occupied by the colonies was determined using an anti -pi I i staining and analyzed using ImageJ software. This total surface was similar with or without isoproterenol.

### 1.16. Expression and purification of MBP-pilin recombinant proteins

Recombinant MBP-pilin fusion proteins were produced as described before (Helaine, 2007, Proc Natl Acad Sci USA. 104,15888-15893). Fragments of pilV and comP genes lacking the region coding for the amino acid residues 1 to 28 of the full-length proteins were amplified by PCR and subcloned in pMA Lp2X vector (New England Biolabs). The resulting plasmids, pMAL-pilV and pMAL-comP, contain in frame the malE gene, which encodes the maltose-binding protein (MBP), followed by the Factor X a protease recognition site and the truncated pilin coding regions. MBP-pilin fusion proteins are directed into *E.coli* periplasm, where disulfide bond formation can occur. These plasmids were transformed in the PAP5198 *E.coli* strain, deleted for the periplasmic enzymes: OmpT Prt and DegP. The fusion proteins were purified on amylose resin (New England Biolabs). The sequence of forward (F) and reverse (R) primers used in the PCR reactions are listed below (restriction sites used for cloning are in parentheses): pi lVp2X F (EcoR1) 5'-GAATTCCGGCGCGTCCGCCTGTCGG-3' (SEQ ID NO: 8) pilVp2XR (Pst1) 5'-CTGCAGTTAGTCGAAGCCGGGGCAGG-3' (SEQ ID NO: 9) comPp2XF (EcoR1) 5'-GAATTCGAGAAAGCAAAGATAAATGC-3' (SEQ ID NO: 10) comPp2XR (Pst1) 5'-CTGCAGCTACTTAAATAACTTGCAGTCC-3' (SEQ ID NO: 11)

### 1.17. Coating of Staphylococcus aureus with MBP-pilin fusion proteins

*S. aureus* (ATCC 25923) expressing specific receptors for the Fc domain of IgG immunoglobulins were used for this experiment. Bacteria from an overnight culture were adjusted to OD600=0.05 and incubated for 2h at 37°C in pre-warmed LB medium. About 10⁸ bacteria were incubated with 2µg of anti-MBP antibody for 20 min at room temperature. Bacteria were then centrifuged at 4000 rpm for 5min, washed with warm LB 3 times, incubated with 2.5µg of each recombinant fusion protein for 20 min at room temperature. After several washes using DMEM medium supplemented with 10% Fetal Calf Serum, bacteria were used in adhesion assays (incubations of 30 min) or lysed in modified RIPA buffer (50mM Tris pH 7.5, 2mM EDTA, 150mM NaCl, 0.1 % (w/v) SDS, 0.5 % (w/v) Na deoxycholate) containing 1X protease inhibitors cocktail for immunoblots (Sigma-Aldrich).

### 1.18. Reverse-PCR

To determine the efficacy of the siRNA -mediated inhibition of the β2AR, total RNA were extracted using a Rneasy Mini Kit (Qiagen) according to the manufacturer protocol. DNA contaminants were removed using Turbo DNAse (Ambion). Equal amounts of RNA from control siRNA- or β2AR siRNA-transfected cells were used as templates for reverse transcription with the Superscript II Reverse transcriptase (Invitrogen) and an oligo-dT primer, following manufacturer instructions. PCR reaction mixs was prepared containing 1XPCR Buffer (Takara), 1µM dNTP Mix (Roche), 1µM of each primer, 0.5 units of Takara Taq polymerase and 5 µL of cDNA. Primers used for β2AR sequence amplification were: (5'- ATTGAGACCCTGTGCGTGAT-3', forward, SEQ ID NO: 12) and (5'-CTGAAAGACCCTGGAGTAGA-3', reverse, SEQ ID NO: 13); primers for GAPDH sequence amplification were: (5'- TTCCTACCCCCAATGTGTCC-3', forward, SEQ ID NO: 14) and (5'-TCTTGCTCAGTGTCCTTGCT-3', reverse, SEQ ID NO: 15). The amplified products were subjected to electrophoresis on 1.5 % agarose gels and stained with ethidium bromide.

### 1.19. Immunoprecipitation and Immunoblotting

For the experiments aimed at determining the efficacy of siRNA-dependent knockdown, whole cell lysate of hCMEC/D3 cells were washed with ice-cold PBS and lysed in modified RIPA buffer containing 1X protease inhibitors cocktail (Sigma-Aldrich). Whole cell lysates were then boiled and analyzed by SD S-PA GE. For immunoprecipitation studies, HEK-293 cells were transfected with appropriate plasmids using lipofectamine 2000. Two days after transfection dishes were lysed in immunoprecipitation buffer (10mM Hepes, 50mM NaCl, 5mM EDTA, 1mM CaCl2, 1mM MgCl2 and 0,5% Triton X100) containing a protease inhibitor cocktail (Sigma-Aldrich), on ice for 1 h. After centrifugation at 13000xg for 20min, the supernatant was collected and protein concentration determined (Bio Rad protein assay). Supernatants were incubated with the anti-myc antibody for 2h at room temperature and then incubated with protein G-Sepharose beads for 1h at 4°C. After centrifugation, the immunobead-bound material was washed 3 times with lysis buffer. The immunoprecipitates were resolved in 10% SDS-PAGE gels. After transfer on nitrocellulose (Optitran BA-S 83, Millipore), blots were probed with the appropriate antibodies. Immunoblots were revealed by luminescence (ECLplus; GE Healthcare Life Sciences).

### EXAMPLE 2: βarrs play an essential role in the formation of ezrin honeycombs under Nm colonies Protocols and experimental procedures

To investigate the role of βarrs in signaling events elicited by the adhesion of *Nm* to endothelial cells, hCMEC/D3 cells, a human brain endothelial cell line, which stably maintains in culture phenotypic features of BBB in culture, was used as a model. Monolayers of hCMEC/D3 cells, inoculated with a capsulated piliated serogroup C *Nm* strain, displayed a characteristic reticulated immunofluorescent ezrin staining (used as a marker of cortical plaques) beneath 90% of bacterial colonies (data not shown). Cortical plaques were also previously reported to contain actin, cortactin, signaling molecules recruited in response to *Nm* adhesion, as well as components of both adherens and tight junctions. In hCMEC/D3 cells expressing green fluorescent protein (GFP)-tagged βarr2 (βarr2-GFP), βarr2-GFP colocalized with ezrin and bacteria, whereas in cells not in contact with *Nm* colonies or in non-infected monolayers βarr2-GFP displayed its expected diffuse cytosolic distribution, indicating that bacterial colonies are capable of locally translocating βarr2. Similar results were obtained in cells expressing βarr1-GFP and for endogenous βarr2 (data not shown). hCMEC/D3 cells, which predominantly express βarr2, were then treated with small interfering RNAs (siRNAs) to establish whether βarr translocation to bacterial colonies occurs upstream or downstream of ezrin recruitment. Reduction of both βarr1 and βarr2 levels, via specific siRNAs, led to a decrease of ezrin-containing cortical plaques in an *Nm* adhesion assay (Figure 1, left panel). The G protein receptor kinase (GRK) family specifically phosphorylates agonist-activated GPCRs, leading to their desensitization. GPCR phosphorylation by GRKs provides docking sites for βarrs. GRK2, a member of this family, was recruited under meningococcal colonies and its depletion inhibited both ezrin and βarr2 translocation under bacteria (Figure 1, left and right panels). Control siRNA or siRNA directed against another signaling molecule, the heterotrimeric Gas protein, did not affect cortical plaque formation (Figure 1, left panel).

Altogether these data indicate that βarr translocation precedes ezrin recruitment, and are consistent with direct or indirect activation of a cell surface GPCR following tfp-mediated adhesion to endothelial cells.

### EXAMPLE 3: Nm adhesion to endothelial cells promotes βarr-biased β2AR signaling

hCMEC/D3 cells naturally express several GPCRs, including β2ARs, angiotensin II AT1 receptors and various chemokine receptors. Sustained stimulation with agonists is known to induce GPCR desensitization and endocytosis, thus reducing their density at the cell surface. We reasoned that, if one of these receptors was activated by *Nm* to mediate βarr and ezrin translocation to *Nm* colonies, specific ligands of the receptor might interfere with this phenomenon. Various agonists were tested using the formation of ezrin honeycombs in hCMEC/D3 cells as readout. Cells were pre-treated before *Nm* inoculation with appropriate agonists for 2h, a condition which often induces internalization and desensitization of cognate receptors. Among the compounds tested at saturating (10xK_{D}) concentrations, (Figure 2, top left panel), only isoproterenol, a β2AR-specific agonist, showed an effect on ezrin recruitment. Moreover, isoproterenol elicited a dose-dependent reduction in the number of ezrin-containing cortical plaques (Figure 2, top right panel). This effect was not due to a direct inhibition by isoproterenol of *Nm* growth in culture or of bacterial aggregation properties (data not shown). Also, isoproterenol pre-treatment did not reduce bacterial adhesion in static conditions (data not shown). In hCMEC/D3 cells expressing β2AR fused to YFP (β2AR-YFP), the formation of *Nm* colonies redistributed the receptor to ezrin-containing honeycombs (data not shown). In contrast, in hCMEC/D3 cells pretreated for 1h with isoproterenol, β2AR-YFP was internalized in perinuclear endosomes and almost excluded from the small areas of residual ezrin below *Nm* colonies. To confirm that the inhibition on ezrin honeycomb formation by isoproterenol was due to β2AR internalization, a similar experiment was conducted in the presence of endocytosis inhibitors (Figure 2, bottom left panel). The first, a specific inhibitor of the clathrin-dependent endocytic machinery, is a dominant negative mutant of Eps15 (Eps15-DIII. The second consists of the isolated carboxyterminal tail of βarr, which contains clathrin and AP2 binding sites and inhibits β2AR endocytosis. Supporting our hypothesis, hCMEC/D3 cells transfected with either mutant displayed a significantly reduced effect of isoproterenol on ezrin recruitment below the *Nm* colonies. The addition during the pre-incubation period of propranolol, a β2AR orthosteric antagonist, which inhibits receptor activation and endocytosis, blocked the effect of isoproterenol, as shown by the number of ezrin honeycombs, which remained comparable to that of untreated cells (Figure 2, bottom right panel). Finally, siRNA directed against the β2AR in hCMEC/D3 cells, significantly decreased both the number of ezrin honeycombs (Figure 2, bottom right panel) and βarr2 recruitment under bacterial colonies (Figure 2, bottom left panel).

The data above indicate that the β2AR is involved in the early signaling events downstream of *Nm* adhesion.

GPCRs display various conformations, which are selectively stabilized, depending on the ligand that activates the receptor. Each conformation being competent for the activation of one or more effectors, different ligands can selectively activate or inhibit different subsets of effectors, leading to different signaling outputs. Activation of β2ARs with catecholamines or isoproterenol, for example, stimulates both the G protein Gαₛ-dependent activation of adenylyl cyclase and the Gas-independent activation of βarrs. Activation of endogenous β2AR on hCMEC/D3 cells elicited a robust stimulation of cAMP production in response to isoproterenol, monitored by a cAMP response element (CRE) luciferase reporter, which was completely blocked by propranolol (data not shown). Incubation of hCMEC/D3 cells with *Nm* at a multiplicity of infection of 100 did not enhance the cAMP-dependent signal. Moreover, since the preincubation of hCMEC/D3 cells with saturating propranolol alone (Figure 2, bottom right panel), or knockdown of Gas by siRNA (data not shown) had no effect on the number of ezrin-containing cortical plaques elicited by *Nm,* the *Nm*-promoted stimulation of β2ARs appears to be biased toward the activation of the βarr pathway.

### EXAMPLE 4: Nm-promoted signaling reconstituted in infection-incompetent cells via the expression of exogenous β2ARs and βarrs

To obtain additional evidence that the β2AR-βarr pathway is critical for the *Nm-*induced formation of cortical plaques in human cells, we investigated whether cells, which are not competent for the adhesion and signaling of *Nm,* can be switched to a permissive phenotype via the expression of exogenous proteins. HEK-293 cells were used as a model because they only express modest concentrations of endogenous βarrs and no β2ARs. Piliated capsulated *Nm* did not adhere onto HEK-293 cells (data not shown) and, consequently did not promote the formation of cortical plaques (data not shown). Transfection of the β2AR and/or βarr constructs in HEK-293 cells did not restore bacterial adhesion and/or signaling (data not shown), thus excluding that the β2AR on its own mediates tfp-dependent adhesion of capsulated *Nm*. However, since tfp-mediated adhesion and signaling are two independent events, the lack of initial bacterial adhesion could be responsible for the absence of signaling. Type IV pili are the only bacterial attributes capable of promoting adhesion of capsulated *Nm*. Yet, non-capsulated (SiaD-) strains can interact with host cells via Opa proteins, which recognize CEACAMs (carcinoembryonic antigen-related cell adhesion molecules), cellular receptors members of the Immunoglobulin superfamily. HEK-293 cells being devoid of CEACAMs, were thus transfected with a plasmid encoding CEACAM-1 and subsequently infected with a non-capsulated (SiaD-) Opa+ derivative of the piliated capsulated serogroup C strain used in the experiments above. Although the expression of CEACAM-1 allowed the development of piliated *Nm* colonies at the surface of HEK-293 cells, colonies did not induce significant recruitment of ezrin (data not shown). HEK-293 cells were then cotransfected with plasmids coding for CEACAM-1 and GFP (used as transfection marker) in the absence or presence of the β2AR and/or βarr2 plasmids, and the recruitment of ezrin assessed in GFP-positive cells. In the absence of exogenous β2AR and βarr2, only a low percentage of colonies recruited ezrin in microvilli-like structures with no visible honeycombs (Figure 3). Transfection of the β2AR plasmid significantly raised the number of colonies (≈30%), which recruited ezrin in GFP-positive cells. The additional expression of exogenous βarr2 boosted the number of ezrin-recruiting colonies to nearly 70%, with typical honeycombs structures being visible in more than 50% of the cases (Figure 3). In addition, the recruitment of ezrin was specifically promoted by the expression of the β2AR, as shown by the absence of ezrin recruitment above control values with expression of the AT1 angiotensin receptor, CCR5 or CXCR4 (Figure 3). Interestingly, 3D reconstruction of confocal immunofluorescence images showed that ezrin accumulates both beneath and between meningococci within cellular protrusions similar to those observed in hCMEC/D3 cells infected with capsulated *Nm* (data not shown). Finally, cortical plaques in HEK-293 cells contained the exogenous β2AR and βarr2 as well as endogenous actin and p120 catenin (data not shown). In order to verify that tfp were responsible for the formation of the cortical plaque, similar experiments were performed using a non-piliated derivative (PilE-) of the non-capsulated Opa+ strain. This strain did not induce the formation of cortical plaques (data not shown).

These data confirm the involvement of the β2AR-βarr pathway in tfp-dependent signaling events, downstream of a β2AR-independent adhesion of *Nm* to host cells.

### EXAMPLE 5: Nm resistance to shear stress requires activation of a β2AR-dependent pathway

Previous studies have shown that the bacteria-induced formation of the cortical plaque and of microvilli-like structures allow *Nm* to adhere to endothelial cells and resist to drag forces generated by bloodstream. To investigate the role of β2AR in the stable adhesion of *Nm* under experimental conditions recapitulating the pathophysiology of bacterial adhesion to brain endothelial cells, hCMEC/D3 cells were grown on IBIDI™ chambers and submitted to laminar flow under an inverted microscope. GFP-expressing bacteria were introduced in the chamber under controlled flow, and the number and size of adhering bacterial colonies counted over time. In control assays, bacterial colonies progressively increased in size, large colonies of 48µm² or more (≥16 bacteria) representing about 50% of the total after 4h, whereas small colonies (≤12µm², about 4 bacteria) accounted for only 20% (Figure 4, right panel). Preincubation of cells with isoproterenol for 1h before the perfusion of bacteria, markedly reduced the average size of *Nm colonies* adhering to endothelial cells under flow: ≈70% of the colonies remained small throughout the experiment, large colonies barely representing 10% (Figure 4, right panel). Interestingly, whereas the number of colonies forming in the absence of flow was similar in control conditions and after isoproterenol pre-treatment (data not shown), under constant flow the β2AR agonist caused a 40% decrease in the number of colonies forming (Figure 4, left panel). These data suggest that, because of inhibited β-adrenergic signaling downstream of *Nm* adhesion, stable bacterial adhesion over time is impaired with larger colonies being progressively "wiped out" by the flow, a hypothesis supported by video data (data not shown).

Thus the activation of β2ARs appears a critical step for *Nm* to remain in strong interaction with brain endothelial cells for a sufficiently long period of time. Consistent with the involvement of a βarr-biased signaling pathway discussed above, pre-incubation with the adenylyl cyclase-stimulating agent forskolin, although inducing marked elevation of cAMP (data not shown) had no significant effect on the number and size of *Nm* colonies compared to control conditions. This result also eliminates the hypothesis of a cAMP-dependent modification of *Nm* adhesion to hCMEC/D3 cells that might have occurred during isoproterenol pre-incubation.

### EXAMPLE 6: Nm crossing of endothelial cell monolayers involves β2ARs

Pili-mediated adhesion of capsulated *Nm* to brain endothelial cells is known to result in the depletion of junctional proteins at the cell-cell interface with a subsequent opening of the intercellular junctions. This depletion is caused by the recruitment of endothelial cell adhesion molecules, such as VE-cadherin, into cortical plaques. We examined the potential role of β2AR activation in these events using hCMEC/D3 cell monolayer grown on transwells as a model. First the number of bacteria passing through the monolayer was counted in control cells and in cells pre-incubated with isoproterenol. The β2AR agonist markedly reduced the total number of meningococci in the lower chamber of transwells (Figure 5, left panel). Gaps induced by *Nm* on cell monolayers were directly visualized by confocal microscopy, and the area of the gaps was quantified using as surface unit the apparent diameter of a bacterium. Pre-incubation with isoproterenol noticeably reduced the number of the gaps of all size (Figure 5, rigth panel).

Together, the above data demonstrate the involvement of the □β2AR in *Nm* crossing of the brain endothelial barrier.

### EXAMPLE 7: Multiple roles of βarrs in cellular signaling events promoted by Nm

The stabilization of bacteria at the cell surface of endothelial cells, which prevents their ejection by blood flow, requires cortactin-dependent formation of actin protrusions. Previous studies have shown that Src-dependent phosphorylation of cortactin is a key signaling intermediate in this context. Src and activated Src (phospho-Src) accumulated below *Nm* colonies in hCMEC/D3 cells (data not shown). Interestingly, it was reported previously that recruitment and activation of Src downstream of β-adrenoceptors can be mediated by βarrs, suggesting that *Nm*-promoted activation of Src may pass via βarrs. Supporting this hypothesis, siRNAs against either the β2AR or βarrs markedly reduced Src recruitment under colonies in hCMEC/D3 cells (Figure 6, top left panel). Docking of Src to βarrs involves the interaction of Src homology 3 (SH3) domains with proline residues of the consensus sequence Pro-X-X-Pro (where X represents any amino acid) within the NH₂-terminus (position 1 to 185) of βarrs. It has been reported that point mutations of these proline residues, lead to a dominant negative form of βarr1 for Src activation, which conserved its capacity of interacting with the β-adrenoceptor and of mediating its endocytosis. Here we constructed the equivalent Src docking mutant in the context of βarr2 (myc-βarr2-ΔSrc or βarr2-ΔSrc-GFP) and examined its effect in *Nm-*promoted phosphorylation of Src and formation of actin-rich cellular protrusions. βarr2-ΔSrc-GFP markedly impaired recruitment of actin under *Nm* colonies in hCMEC/D3 cells, compared to control cells expressing GFP (Figure 6, top middle panel). In addition, analysis of fluorescence images along the z-axis confirmed the marked reduction of actin-rich protrusions among bacteria (data not shown). Immunoblot experiments showed a reduction of phosphorylated Src after *Nm* infection in hCMEC/D3 cells expressing the dominant negative myc-βarr2-ΔSrc, compared to cells expressing myc-βarr2 (data not shown). The dominant negative effect of βarr2-ΔSrc was confirmed in hCMEC/D3 cells under flow. Expression of myc-βarr2-ΔSrc displayed the same effect on *Nm* colony size as the preincubation with isoproterenol (compare Figure 6, top right panel and Figure 4, right panel). βarrs might also participate in later events of *Nm* infection, namely the opening of gaps in the endothelial cell barrier.

This opening has been shown to involve the recruitment of junctional adhesion molecules, such as VE-cadherin and p120-catenin, into the cortical plaques, and their parallel depletion from endothelial cell junctions. We documented the interaction of βarrs with VE-cadherin and p120-catenin by co-immunoprecipitation experiments in both transfected HEK-293 cells and untransfected hCMEC/D3 (data not shown). In addition, the reduction of endogenous βarrs with specific siRNAs markedly and simultaneously affected the recruitment of VE-cadherin, p120-catenin, F-actin and ezrin into cortical plaques (Figure 6, bottom panel), supporting the hypothesis that β2AR-mediated activation of βarrs provides a molecular connection between *Nm* colonies and the recruitment of junctional molecules.

### EXAMPLE 8: Molecular determinants involved in Nm -β2AR interaction

Among the various GPCRs expressed in hCMEC/D3 cells, only the β2AR seems capable of mediating *Nm* signaling, implying some selective interaction between bacterial molecular determinants and a corresponding binding site on the β2AR. The fact that tfp are necessary for reconstituting signaling events in HEK-293 cells (Figure 3) indicates that the putative β2AR "ligand" is present on these structures. Also in favor of this hypothesis are the consequences of the deletion of PilV, a supposed minor pilin component, which has been shown to impair the formation of the cortical plaque (Mikaty et al., 2009, PLoS Pathog 5, e1 000314), a finding confirmed in reconstituted HEK-293 cells (Figure 7, top left panel).

To further characterize the supposed β2AR "ligand" at a molecular level, purified maltose binding protein (MBP)-fused pilins were bound to staphylococci coated with anti-MBP antibodies (Figure 7, top right panel). In addition to PilE, two putative minor pilins, PilV and ComP, were tested. Whereas staphylococci coated with MBP alone or MBP fused to ComP were ineffective, staphylococci carrying either MBP-PilE or MBP-PilV promoted β2AR and βarr2 accumulation under staphylococcal aggregates (Figure 7, top right panel). In control experiments, the AT1 R was not recruited to bacteria. These data suggest that PilE and PilV can function as β2AR "ligands".

Since *Nm* interaction with the β2AR does not involve the natural receptor binding-site (data in Figure 2), the large β2AR extracellular N-terminus appears as a likely candidate region for receptor interaction with the pathogen. To test this hypothesis, we constructed a chimeric Nterβ2AR-AT1R receptor, in which the N-terminus of the β2AR replaced the equivalent portion of the incompetent AT1 R. The chimeric receptor was indeed capable of mediating *Nm*-dependent signaling in the HEK-293 reconstituted model (Figure 7, bottom panel), as did the β2AR. Altogether, these data suggest that pili components can directly interact with the N-terminus of the β2AR.

In addition to conserved biological functions, such as surface mobility, adhesion or microcolony formation onto specific cell types, structural comparisons of the type IV pilins from different type of Gram-negative bacteria showed that all type IV pilins, despite some variably structured domains, share similar overall architecture (Craig et al., 2004, Nat Rev Microbiol 2, 363-378). We therefore examined whether *Neisseria gonorrohae* (*Ng*), a close relative of *Nm* which most often cause isolated infection of the genito-urinary tract but can, in rare cases, spread into the bloodstream and colonize meninges (Martín et al., 2008, Emerg Infect Dis 14, 672-674), might elicit similar signaling events to *Nm* in endothelial cells. The principal signaling events reported here for *Nm,* were also found in hCMEC/D3 cells infected by gonococci, demonstrating that the capacity of recruiting β-arrs via β2AR is not exclusive for *Nm* species (data not shown).

### EXAMPLE 9: Discussion

From our data, a more complete picture emerges of the signaling events elicited by *Nm* adhesion to human endothelial cells, leading to stable interaction with host cells and subsequent crossing of the endothelial monolayer. After binding onto target cells, *Nm* tfp interact with and activate the host cell β2AR, leading to the subsequent recruitment of GRK2 and βarrs, independently of the activation of the heterotrimeric Gas protein, and of its downstream adenylyl cyclase-cAMP pathway. The selective activation of part of the signaling pathways downstream of a given GPCR, has been theorized and subsequently confirmed experimentally for synthetic ligands (Violin and Lefkowitz, 2007, Trends Pharmacol Sci 28, 416-422). However, this property, known as biased agonism, had not been described previously in a pathophysiological context.

Once translocated to β2ARs, βarrs mediate at least 2 major events facilitating *Nm* infection. First, they dock and activate Src, leading to the formation of actin-rich cellular protrusions, which protect bacterial colonies against drag forces generated by the bloodstream. Second they provide sustained scaffolding for signaling events under Nm-activated β2ARs, which lead to the accumulation of βarr-interacting proteins, such as VE-cadherin and p120-catenin, into cortical plates under bacterial colonies. This accumulation eventually depletes intercellular junctions, which loosen and become permeable to bacteria. Although epithelial cells are the main target of Ng, gonococci possibly use the same pathway to infect brain endothelial cells *in vivo* in the rare cases of blood dissemination. It remains to be established whether other tfp-bearing bacteria capable of forming microcolonies at the surface of endothelial cells and crossing endothelial monolayers, can also hijack βarrs and/or β2ARs.

A few other examples of aberrant use of cellular GPCRs by pathogens to infect host cells exist, but the molecular mechanisms involved so far are quite different from those described here. For instance, *Streptococcus pneumoniae* is co-internalized with the platelet-activating factor receptor PAFr and delivered via transcytosis to the opposite basolateral membrane of endothelial cells. Also, the Human Immunodeficiency Virus, HIV, requires the association with CXCR4 or CCR5 chemokine receptors to infect cells. Binding of the viral gp120 to chemokine receptors, induces unmasking and insertion of a fusion peptide into the target cell membrane. Before interacting with chemokine receptors, HIV needs to bind to a second receptor, CD4.

Our experiments have shown that both sustained adhesion of *Nm* to endothelial cells under controlled flow and their crossing of endothelial cell monolayers by opening cellular junctions are markedly inhibited by pharmacological pre-activation and internalization of β2ARs. Thus, β2AR ligands, which induce β2AR endocytosis, might be beneficial in association with antibiotics to prevent or treat meningococcal infection.

In conclusion, signaling downstream of *Nm* adhesion to human brain endothelial cells, involves a biased activation of β2ARs and the subsequent βarr-dependent docking and activation of Src, which in turn activate the cortactin-dependent formation of cellular protrusions. Subsequently, βarrs scaffold cytoskeletal and junctional proteins under stabilized bacterial colonies, which progressively weakens the endothelial barrier until anatomical breakage occurs. This scenario represents a novel mechanism by which a pathogen hijacks cellular signaling machineries to infect target tissues. Fortuitously, the signaling pathway involved in this scenario can be targeted by clinically approved drugs, which could now be tested as adjuvant therapies, not only in the context of *Nm* meningitis but also possibly for meningococcal sepsis, the most devastating form of *Nm* infection, which is characterized by diffuse endothelial leakage and subsequent irreversible shock.

## Claims

1. A protein that comprises or consists of:
(a) the meningococcal PiIV sequence SEQ ID NO: 2 or a sequence at least 80% identical to SEQ ID NO:2, or
(b) an immunogenic fragment of (a) that comprises or consists of SEQ ID N°21, or
(c) an immunogenic fragment of (a) that comprises or consists of a sequence that is at least 80% identical to SEQ ID NO: 21, or
(d) an immunogenic fragment of (a) that comprises or consists of SEQ ID N°19, or
(e) an immunogenic fragment of (a) that comprises or consists of a sequence that is at least 80% identical to SEQ ID N°19;
for use as a vaccine for preventing the spreading of meningococci into the meningeal space, wherein said vaccine induces the production of protective antibodies inhibiting the interaction between PiIV and mammalian β2 adrenoreceptor.

2. A combination of at least two of:
a) a protein that comprises or consists of :
(i) the meningococcal PiIV sequence SEQ ID NO: 2 or a sequence at least 80% identical to SEQ ID NO:2, or
(ii) an immunogenic fragment of (i) that comprises or consists of SEQ ID N°21, or
(iii) an immunogenic fragment of (i) that comprises or consists of a sequence that is at least 80% identical to SEQ ID NO: 21, or
(iv) an immunogenic fragment of (i) that comprises or consists of SEQ ID N°19, or
(v) an immunogenic fragment of (i) that comprises or consists of a sequence that is at least 80% identical to SEQ ID N°19; and
b) a meningococcal vaccine antigen;
for simultaneous or sequential use as a vaccine for preventing the spreading of meningococci into the meningeal space, wherein said vaccine induces the production of protective antibodies inhibiting the interaction between PiIV and mammalian β2 adrenoreceptor, wherein said meningococcal vaccine antigen is not a PilE, PilV, or an immunogenic fragment thereof.

3. A fusion protein comprising at least two of:
a) a protein that comprises or consists of :
(i) the meningococcal PiIV sequence SEQ ID NO: 2 or a sequence at least 80% identical to SEQ ID NO:2, or
(ii) an immunogenic fragment of (i) that comprises or consists of SEQ ID N°21, or
(iii) an immunogenic fragment of (i) that comprises or consists of a sequence that is at least 80% identical to SEQ ID NO: 21, or
(iv) an immunogenic fragment of (i) that comprises or consists of SEQ ID N°19, or
(v) an immunogenic fragment of (i) that comprises or consists of a sequence that is at least 80% identical to SEQ ID N°19; and
b) a meningococcal vaccine antigen,
wherein said meningococcal vaccine antigen is not PilE, PiIV, or an immunogenic fragment thereof.

4. A fusion protein according to claim 3 for use as a vaccine for preventing the spreading of meningococci into the meningeal space, wherein said vaccine induces the production of protective antibodies inhibiting the interaction between PiIV and mammalian β2 adrenoreceptor, wherein said meningococcal vaccine antigen is not PilE, PiIV, or an immunogenic fragment thereof.

5. A vaccine composition or an immunogenic composition for use for preventing the spreading of meningococci into the meningeal space by the production of protective antibodies inhibiting the interaction between PiIV and mammalian β2 adrenoreceptor, wherein said vaccine or immunogenic composition comprises or consists of:
- a protein that comprises or consists of :
a) the meningococcal PiIV sequence SEQ ID NO: 2 or a sequence at least 80% identical to SEQ ID NO:2, or
b) an immunogenic fragment of (a) that comprises or consists of SEQ ID N°21, or
c) an immunogenic fragment of (a) that comprises or consists of a sequence that is at least 80% identical to SEQ ID NO: 21, or
d) an immunogenic fragment of (a) that comprises or consists of SEQ ID N°19, or
e) an immunogenic fragment of (a) that comprises or consists of a sequence that is at least 80% identical to SEQ ID N°19;
- a combination of at least two of:
a) a protein that comprises or consists of :
(i) the meningococcal PiIV sequence SEQ ID NO: 2 or a sequence at least 80% identical to SEQ ID NO:2, or
(ii) an immunogenic fragment of (i) that comprises or consists of SEQ ID N°21, or
(iii) an immunogenic fragment of (i) that comprises or consists of a sequence that is at least 80% identical to SEQ ID NO: 21, or
(iv) an immunogenic fragment of (i) that comprises or consists of SEQ ID N°19, or
(v) an immunogenic fragment of (i) that comprises or consists of a sequence that is at least 80% identical to SEQ ID N°19; and
b) a meningococcal vaccine antigen; or
- a fusion protein according to claim 3.

6. A protein for the use according to claim 1, a combination for the use according to claim 2, a fusion protein according to claim 3, a fusion protein for the use according to claim 4 or a vaccine or an immunogenic composition for the use according to claim 5, wherein said immunogenic fragment does not carry the hydrophobic domain of sequence SEQ ID NO: 17.

## Patentansprüche

1. Ein Protein, das:
(a) die Meningokokken-PilV-Sequenz SEQ ID NO:2 oder eine Sequenz, die zu mindestens 80 % identisch ist zu SEQ ID NO:2, oder
(b) ein immunogenes Fragment von (a), das SEQ ID NO:21 umfasst oder daraus besteht, oder
(c) ein immunogenes Fragment von (a), das eine Sequenz, die zu mindestens 80 % identisch ist zu SEQ ID NO:21, umfasst oder daraus besteht, oder
(d) ein immunogenes Fragment von (a), das SEQ ID NO:19 umfasst oder daraus besteht, oder
(e) ein immunogenes Fragment von (a), das eine Sequenz, die zu mindestens 80 % identisch ist zu SEQ ID NO:19, umfasst oder daraus besteht,
umfasst oder daraus besteht;
zur Verwendung als ein Impfstoff zur Verhinderung der Ausbreitung von Meningokokken in den meningealen Raum, wobei der Impfstoff die Produktion von schützenden Antikörpern induziert, die die Interaktion zwischen PiIV und Säugetier-ß2-Adrenorezeptoren inhibiert.

2. Eine Kombination von mindestens zwei von:
a) einem Protein, das:
(i) die Meningokokken-PilV-Sequenz SEQ ID NO:2 oder eine Sequenz, die zu mindestens 80 % identisch ist zu SEQ ID NO:2, oder
(ii) ein immunogenes Fragment von (i), das SEQ ID NO:21 umfasst oder daraus besteht, oder
(iii) ein immunogenes Fragment von (i), das eine Sequenz, die zu mindestens 80 % identisch ist zu SEQ ID NO:21, umfasst oder daraus besteht, oder
(iv) ein immunogenes Fragment von (i), das SEQ ID NO:19 umfasst oder daraus besteht, oder
(v) ein immunogenes Fragment von (i), das eine Sequenz, die zu mindestens 80 % identisch ist zu SEQ ID NO:19, umfasst oder daraus besteht,
umfasst oder daraus besteht; und
b) einem Meningokokken-Impfstoff-Antigen;
zur simultanen oder sequentiellen Verwendung als ein Impfstoff zur Verhinderung der Ausbreitung von Meningokokken in den meningealen Raum, wobei der Impfstoff die Produktion von schützenden Antikörpern induziert, die die Interaktion zwischen PilV und Säugetier-ß2-Adrenorezeptoren inhibiert, wobei das Meningokokken-Impfstoff-Antigen nicht PilE, PiIV oder ein immunogenes Fragment davon ist.

3. Ein Fusionsprotein, das mindestens zwei von:
a) einem Protein, das:
(i) die Meningokokken-PiIV-Sequenz SEQ ID NO:2 oder eine Sequenz, die zu mindestens 80 % identisch ist zu SEQ ID NO:2, oder
(ii) ein immunogenes Fragment von (i), das SEQ ID NO:21 umfasst oder daraus besteht, oder
(iii) ein immunogenes Fragment von (i), das eine Sequenz, die zu mindestens 80 % identisch ist zu SEQ ID NO:21, umfasst oder daraus besteht, oder
(iv) ein immunogenes Fragment von (i), das SEQ ID NO:19 umfasst oder daraus besteht, oder
(v) ein immunogenes Fragment von (i), das eine Sequenz, die zu mindestens 80 % identisch ist zu SEQ ID NO:19, umfasst oder daraus besteht,
umfasst oder daraus besteht; und
b) einem Meningokokken-Impfstoff-Antigen
umfasst oder daraus besteht,
wobei das Meningokokken-Impfstoff-Antigen nicht PilE, PilV oder ein immunogenes Fragment davon ist.

4. Ein Fusionsprotein gemäß Anspruch 3 zur Verwendung als ein Impfstoff zur Verhinderung der Ausbreitung von Meningokokken in den meningealen Raum, wobei der Impfstoff die Produktion von schützenden Antikörpern induziert, die die Interaktion zwischen PiIV und Säugetier-ß2-Adrenorezeptoren inhibiert, wobei das Meningokokken-Impfstoff-Antigen nicht PilE, PilV oder ein immunogenes Fragment davon ist.

5. Eine Impfstoffzusammensetzung oder eine immunogene Zusammensetzung zur Verwendung zur Verhinderung der Ausbreitung von Meningokokken in den meningealen Raum durch die Produktion von schützenden Antikörpern induziert, die die Interaktion zwischen PilV und Säugetier-ß2-Adrenorezeptoren inhibiert, wobei der Impfstoff oder die immunogene Zusammensetzung:
- ein Protein, das:
a) die Meningokokken-PilV-Sequenz SEQ ID NO:2 oder eine Sequenz, die zu mindestens 80 % identisch ist zu SEQ ID NO:2, oder
b) ein immunogenes Fragment von (a), das SEQ ID NO:21 umfasst oder daraus besteht, oder
c) ein immunogenes Fragment von (a), das eine Sequenz, die zu mindestens 80 % identisch ist zu SEQ ID NO:21, umfasst oder daraus besteht, oder
d) ein immunogenes Fragment von (a), das SEQ ID NO:19 umfasst oder daraus besteht, oder
e) ein immunogenes Fragment von (a), das eine Sequenz, die zu mindestens 80 % identisch ist zu SEQ ID NO:19, umfasst oder daraus besteht,
umfasst oder daraus besteht;
- eine Kombination von mindestens zwei von:
a) einem Protein, das:
(i) die Meningokokken-PilV-Sequenz SEQ ID NO:2 oder eine Sequenz, die zu mindestens 80 % identisch ist zu SEQ ID NO:2, oder
(ii) ein immunogenes Fragment von (i), das SEQ ID NO:21 umfasst oder daraus besteht, oder
(iii) ein immunogenes Fragment von (i), das eine Sequenz, die zu mindestens 80 % identisch ist zu SEQ ID NO:21, umfasst oder daraus besteht, oder
(iv) ein immunogenes Fragment von (i), das SEQ ID NO:19 umfasst oder daraus besteht, oder
(v) ein immunogenes Fragment von (i), das eine Sequenz, die zu mindestens 80 % identisch ist zu SEQ ID NO:19, umfasst oder daraus besteht,
b) einem Meningokokken-Impfstoff-Antigen; oder
- ein Fusionsprotein gemäß Anspruch 3
umfasst oder daraus besteht; und
umfasst oder daraus besteht.

6. Ein Protein zur Verwendung gemäß Anspruch 1, eine Kombination zur Verwendung gemäß Anspruch 2, ein Fusionsprotein gemäß Anspruch 3, ein Fusionsprotein zur Verwendung gemäß Anspruch 4 oder ein Impfstoff oder eine immunogene Zusammensetzung zur Verwendung gemäß Anspruch 5, wobei das immunogene Fragment nicht die hydrophobe Domäne der Sequenz SEQ ID NO:17 trägt.

## Revendications

1. Protéine qui comprend ou consiste en :
(a) la séquence de la PiIV à méningocoque SEQ ID NO : 2 ou une séquence au moins 80 % identique à la SEQ ID NO : 2, ou
(b) un fragment immunogène de (a) qui comprend ou consiste en la SEQ ID N° 21, ou
(c) un fragment immunogène de (a) qui comprend ou consiste en une séquence qui est au moins 80 % identique à la SEQ ID NO : 21, ou
(d) un fragment immunogène de (a) qui comprend ou consiste en la SEQ ID N° 19, ou
(e) un fragment immunogène de (a) qui comprend ou consiste en une séquence qui est au moins 80 % identique à la SEQ ID N° 19 ;
pour utilisation en tant que vaccin pour la prévention de la prolifération des méningocoques dans l'espace méningé, dans laquelle ledit vaccin induit la production d'anticorps protecteurs inhibant l'interaction entre la PiIV et un récepteur β2 adrénergique de mammifère.

2. Combinaison d'au moins deux parmi :
a) une protéine qui comprend ou consiste en :
(i) la séquence de la PilV à méningocoque SEQ ID NO : 2 ou une séquence au moins 80 % identique à la SEQ ID NO : 2, ou
(ii) un fragment immunogène de (i) qui comprend ou consiste en la SEQ ID N° 21, ou
(iii) un fragment immunogène de (i) qui comprend ou consiste en une séquence qui est au moins 80 % identique à la SEQ ID NO : 21, ou
(iv) un fragment immunogène de (i) qui comprend ou consiste en la SEQ ID N° 19, ou
(v) un fragment immunogène de (i) qui comprend ou consiste en une séquence qui est au moins 80 % identique à la SEQ ID N° 19 ; et
b) un antigène vaccinant anti-méningococcique ;
pour utilisation simultanément ou séquentielle en tant que vaccin pour la prévention de la prolifération des méningocoques dans l'espace méningé, dans laquelle ledit vaccin induit la production d'anticorps protecteurs inhibant l'interaction entre la PilV et un récepteur β2 adrénergique de mammifère, dans laquelle ledit antigène vaccinant anti-méningococcique n'est pas une PilE, une PilV, ou un fragment immunogène de celles-ci.

3. Protéine hybride comprenant au moins deux parmi :
a) une protéine qui comprend ou consiste en :
(i) la séquence de la PilV à méningocoque SEQ ID NO : 2 ou une séquence au moins 80 % identique à la SEQ ID NO : 2, ou
(ii) un fragment immunogène de (i) qui comprend ou consiste en la SEQ ID N° 21, ou
(iii) un fragment immunogène de (i) qui comprend ou consiste en une séquence qui est au moins 80 % identique à la SEQ ID NO : 21, ou
(iv) un fragment immunogène de (i) qui comprend ou consiste en la SEQ ID N° 19, ou
(v) un fragment immunogène de (i) qui comprend ou consiste en une séquence qui est au moins 80 % identique à la SEQ ID N° 19 ; et
b) un antigène vaccinant anti-méningococcique,
dans laquelle ledit antigène vaccinant anti-méningococcique n'est pas une PilE, une PiIV, ou un fragment immunogène de celles-ci.

4. Protéine hybride selon la revendication 3 pour utilisation en tant que vaccin pour la prévention de la prolifération des méningocoques dans l'espace méningé, dans laquelle ledit vaccin induit la production d'anticorps protecteurs inhibant l'interaction entre la PilV et un récepteur β2 adrénergique de mammifère, dans laquelle ledit antigène vaccinant anti-méningococcique n'est pas une PilE, une PilV, ou un fragment immunogène de celles-ci.

5. Composition vaccinale ou composition immunogène pour utilisation dans la prévention de la prolifération des méningocoques dans l'espace méningé par la production d'anticorps protecteurs inhibant l'interaction entre la PilV et un récepteur β2 adrénergique de mammifère, dans laquelle ladite composition vaccinale ou immunogène comprend ou consiste en :
- une protéine qui comprend ou consiste en :
a) la séquence de la PiIV à méningocoque SEQ ID NO : 2 ou une séquence au moins 80 % identique à la SEQ ID NO : 2, ou
b) un fragment immunogène de (a) qui comprend ou consiste en la SEQ ID N° 21, ou
c) un fragment immunogène de (a) qui comprend ou consiste en une séquence qui est au moins 80 % identique à la SEQ ID NO : 21, ou
d) un fragment immunogène de (a) qui comprend ou consiste en la SEQ ID N° 19, ou
e) un fragment immunogène de (a) qui comprend ou consiste en une séquence qui est au moins 80 % identique à la SEQ ID N° 19 ;
- une combinaison d'au moins deux parmi :
a) une protéine qui comprend ou consiste en :
(i) la séquence de la PilV à méningocoque SEQ ID NO : 2 ou une séquence au moins 80 % identique à la SEQ ID NO : 2, ou
(ii) un fragment immunogène de (i) qui comprend ou consiste en la SEQ ID N° 21, ou
(iii) un fragment immunogène de (i) qui comprend ou consiste en une séquence qui est au moins 80 % identique à la SEQ ID NO : 21, ou
(iv) un fragment immunogène de (i) qui comprend ou consiste en la SEQ ID N° 19, ou
(v) un fragment immunogène de (i) qui comprend ou consiste en une séquence qui est au moins 80 % identique à la SEQ ID N° 19 ; et
b) un antigène vaccinant anti-méningococcique ; ou
- une protéine hybride selon la revendication 3.

6. Protéine pour utilisation selon la revendication 1, combinaison pour utilisation selon la revendication 2, protéine hybride selon la revendication 3, protéine hybride pour utilisation selon la revendication 4 ou composition vaccinale ou immunogène pour utilisation selon la revendication 5, dans laquelle ledit fragment immunogène ne porte pas le domaine hydrophobe de la séquence SEQ ID NO : 17.
